(19) Europäisches Patentamt
European Patent Office
Office européen des brevets

(11) Veröffentlichungsnummer: **0 262 087**
**A1**

(12) # EUROPÄISCHE PATENTANMELDUNG

(21) Anmeldenummer: **87810498.3**

(22) Anmeldetag: **31.08.87**

(51) Int. Cl.[4]: **C 07 D 277/34**
C 07 D 233/70,
C 07 D 285/12,
C 07 D 249/12,
C 07 D 271/10,
C 07 D 285/08,
C 07 D 277/68, A 01 N 43/78,
A 01 N 43/76, A 01 N 43/82,
A 01 N 43/653

(30) Priorität: **04.09.86 CH 3561/86**

(43) Veröffentlichungstag der Anmeldung:
**30.03.88 Patentblatt 88/13**

(84) Benannte Vertragsstaaten:
**AT BE CH DE ES FR GB GR IT LI NL**

(71) Anmelder: **CIBA-GEIGY AG**
**Klybeckstrasse 141**
**CH-4002 Basel (CH)**

(72) Erfinder: **Ehrenfreund, Josef, Dr.**
**Amselstrasse 11**
**CH-4123 Allschwil (CH)**

**Böger, Manfred**
**Wilhelm Glockstrasse 14**
**D-7858 Weil am Rhein 5 (DE)**

**Kristiansen, Odd, Dr.**
**Delligrabenstrasse 7**
**CH-4313 Möhlin (CH)**

**Drabek, Jozef, Dr.**
**Benkenstrasse 12**
**CH-4104 Oberwil (CH)**

**Kristinsson, Haukur, Dr.**
**Leimenstrasse 30**
**CH-4051 Basel (CH)**

(54) **Substituierte Thioharnstoffe, Isothioharnstoffe und Carbodiimide.**

(57) Die Erfindung betrifft neue substituierte Phenylthioharnstoffe, Phenylisothioharnstoffe und Phenylcarbodiimide der Formel I

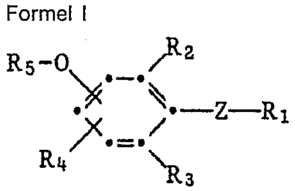

(I),

worin

Z 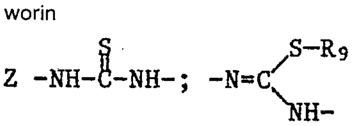 ; oder -N=C=N-;

$R_1$ $C_1$-$C_{12}$-Alkyl; $C_3$-$C_{10}$-Alkenyl; $C_3$-$C_{10}$-Alkinyl; $C_3$-$C_8$-Cycloalkyl; Mono- oder Di-($C_3$-$C_6$-cycloalkyl)-$C_1$-$C_3$-Alkyl; Alkoxyalkyl mit insgesamt 3-10 C-Atomen, halogensubstituiertes $C_1$-$C_{12}$-Alkyl; polycyclisches $C_7$-$C_{10}$-Cycloalkyl; oder Phenyl-$C_1$-$C_5$-alkyl, gegebenenfalls im Phenylteil bis zu zweifach gleich oder verschieden mit Halogen, Methoxy, Ethoxy oder $C_1$-$C_5$-Alkyl substituiert;

$R_2$ Wasserstoff; oder $C_1$-$C_5$-Alkyl;

$R_3$ $C_1$-$C_5$-Alkyl; oder $C_3$-$C_6$-Cycloalkyl;

$R_4$ Wasserstoff; oder $C_1$-$C_4$-Alkyl;

$R_5$ einen 5-gliedrigen heterocyclischen Rest mit 1-4 Heteroatomen der Gruppe N,O,S, welcher bis zu dreifach an den Kohlenstoff-bzw. Stickstoffatomen durch die Reste $R_6$, $R_7$, $R_8$ substituiert sein kann; oder einen benzokondensierten 5-gliedrigen hetero cyclischen Rest mit 1-7 Heteroatomen der Gruppe N,O,S, welcher bis zu dreifach an den Kohlenstoff- bzw. Stickstoffatomen durch die Reste $R_6$, $R_7$, $R_8$ substituiert sein kann;

$R_6$ und $R_7$ unabhängig voneinander Wasserstoff; Halogen; $C_1$-$C_4$-Alkyl; halogensubstituiertes $C_1$-$C_4$-Alkyl; $C_1$-$C_4$-Alkoxy; halogensubstituiertes $C_1$-$C_4$-Alkoxy; Phenyl; oder $C_1$-$C_4$-Alkylthio;

$R_8$ Wasserstoff; oder $C_1$-$C_4$-Alkyl;

$R_9$ $C_1$-$C_5$-Alkyl; $C_2$-$C_5$-Alkenyl; $C_3$-$C_4$-Alkinyl; Alkoxyalkyl mit insgesamt bis zu 10 C-Atomen; oder Alkylthioalkyl mit insgesamt bis zu 10 C-Atomen

bedeuten; sowie deren Salze mit Säuren und Basen. Verfahren zur Herstellung dieser Verbindungen, ihrer Verwendung und in der Schädlingsbekämpfung und neue Zwischenprodukte.

EP 0 262 087 A1

## Beschreibung

### Substituierte Thioharnstoffe, Isothioharnstoffe und Carbodiimide

Die vorliegende Erfindung betrifft neue, durch fünfgliedrige heterocyclische Reste substituierte Thioharnstoffe, Isothioharnstoffe sowie Carbodiimide, Verfahren zu ihrer Herstellung und ihre Verwendung in der Schädlingsbekämpfung.

Insektizid und akarizid wirksame N-(Phenoxyphenyl)-N′-alkyl-thioharnstoffe und -isothioharnstoffe sind bereits aus der britischen Patentanmeldung Nr. 2.060.626 und der europäischen Patentschrift Nr. 0.025.010 bekannt. Entsprechende N-Pyridyloxyphenyl-N′-tert.-butyl-thioharnstoffe und - isothioharnstoffe sind Gegenstand der japanischen Patentveröffentlichung Nr. 58-79979. Ferner werden N-Pyridyloxyphenyl-N′-alkyl-carbodiimide mit insektizider und akarizider Wirkung in der europäischen Patentanmeldung Nr. 0.175.649 beschrieben. Demgegenüber unterscheiden sich die erfindungsgemässen Thioharnstoffe, Isothioharnstoffe und Carbodiimide der Formel I strukturell im wesentlichen durch eine N-Phenylgruppe, die als Substituenten einen über ein Sauerstoffatom gebundenen heterocyclischen Fünfring aufweist. Die bekannten vorstehend erwähnten Verbindungstypen befriedigen nicht immer im Hinblick auf Wirkungsdauer, Wirkungsstärke und Wirkungsbreite. Es wurde nun überraschenderweise gefunden, dass die erfindungsgemässen Verbindungen der Formel I ausgezeichnet als Schädlingsbekämpfungsmittel geeignet sind.

Die erfindungsgemässen Verbindungen haben die Formel I

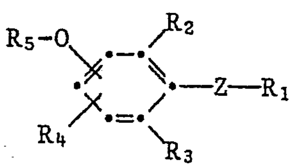 (I),

worin

Z 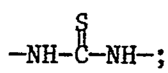; 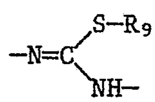 oder $-N=C=N-$;

$R_1$ $C_1$-$C_{12}$-Alkyl; $C_3$-$C_{10}$-Alkenyl; $C_3$-$C_{10}$-Alkinyl; $C_3$-$C_8$-Cycloalkyl; mit 1 oder 2 $C_3$-$C_6$-Cycloalkylresten substituiertes $C_1$-$C_3$-Alkyl; Alkoxyalkyl mit insgesamt 3-10 C-Atomen; halogensubstituiertes $C_1$-$C_{12}$-Alkyl; polycyclisches $C_7$-$C_{10}$-Cycloalkyl; oder Phenyl-$C_1$-$C_5$-alkyl, gegebenenfalls im Phenylteil bis zu zweifach gleich oder verschieden mit Halogen, Methoxy, Aethoxy oder $C_1$-$C_5$-Alkyl substituiert;
$R_2$ Wasserstoff; oder $C_1$-$C_5$-Alkyl;
$R_3$ $C_1$-$C_5$-Alkyl; oder $C_3$-$C_6$-Cycloalkyl;
$R_4$ Wasserstoff; oder $C_1$-$C_4$-Alkyl;
$R_5$ einen 5-gliedrigen heterozyclischen Rest mit 1-4 Heteroatomen der Gruppe N,O,S, welcher bis zu dreifach an den Kohlenstoff-bzw. Stickstoffatomen durch die Reste $R_6$, $R_7$, $R_8$ substituiert sein kann; oder einen benzokondensierten 5-gliedrigen heterozyclischen Rest mit 1-2 Heteroatomen der Gruppe N,O,S, welcher bis zu dreifach an den Kohlenstoff- bzw. Stickstoffatomen durch die Reste $R_6$, $R_7$, $R_8$ substituiert sein kann;
$R_6$ und $R_7$ unabhängig voneinander Wasserstoff; Halogen; $C_1$-$C_4$-Alkyl; halogensubstituiertes $C_1$-$C_4$-Alkyl; $C_1$-$C_4$-Alkoxy; halogensubstituiertes $C_1$-$C_4$-Alkoxy; Phenyl; oder $C_1$-$C_4$-Alkylthio; $R_8$ Wasserstoff; oder $C_1$-$C_4$-Alkyl;
$R_9$ $C_1$-$C_5$-Alkyl; $C_2$-$C_5$-Alkenyl; $C_3$-$C_4$-Alkinyl; Alkoxyalkyl mit insgesamt 10 C-Atomen; oder Alkylthioalkyl mit insgesamt 10 C-Atomen
bedeuten.

Des weiteren betrifft die Erfindung auch Salze von Verbindungen der Formel I mit geeigneten Säuren und Basen.

In der vorliegenden Beschreibung werden einzelne Vertreter der in den erfindungsgemässen Verbindungen enthaltenen fünfgliedrigen heterozyclischen Reste durch einen Kurzcode benannt. Im einzelnen steht
$Q_1$ für Thiazolyl, $Q_2$ für 1,2,4-Triazolyl,
$Q_3$ für 1,3,4-Thiadiazolyl, $Q_4$ für 1,2,4-Thiadiazolyl,
$Q_5$ für 1,3,4-Oxadiazolyl, $Q_6$ für Thienyl,
$Q_7$ für Benzoxazolyl, $Q_8$ für Benzthiazolyl,
$Q_9$ für Imidazolyl, $Q_{10}$ für Furanyl, $Q_{11}$ für Pyrrolyl,
$Q_{12}$ für Pyrazolyl, $Q_{13}$ für Oxazolyl, $Q_{14}$ für Benzofuranyl,
$Q_{15}$ für Indolyl, $Q_{16}$ für Benzimidazolyl und $Q_{17}$ für Tetrazolyl.

Hervorzuheben sind erfindungsgemäss Verbindungen der Formel I, in der die Reste $R_1$ bis $R_4$, Z und $R_6$ bis $R_9$ wie zuvor definiert sind und in denen der Substituent $R_5$ für nachfolgende heterocyclische Reste steht:

$Q_1$ = 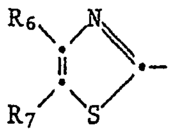 ; $Q_2$ = 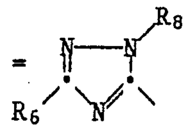 ; $Q_3$ = 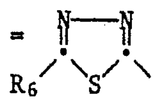 ;

$Q_4$ = 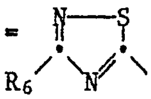 ; $Q_5$ = 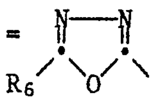 ; $Q_6$ = 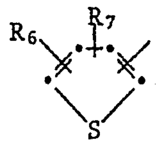 ;

$Q_7$ = 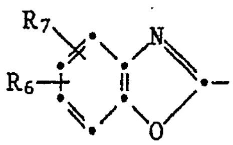 $Q_8$ = 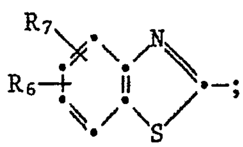 ; $Q_9$ = 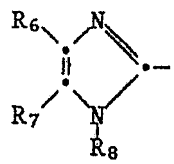 ;

$Q_{10}$ = 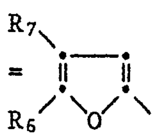 ; $Q_{11}$ = 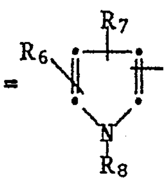 ; $Q_{12}$ = 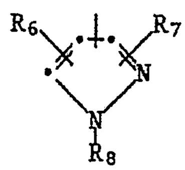 ;

$Q_{13}$ = 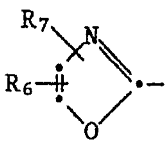 ; $Q_{14}$ = 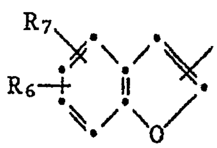 ; $Q_{15}$ = 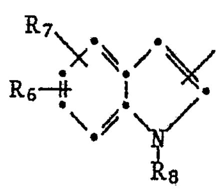 ;

$Q_{16}$ = 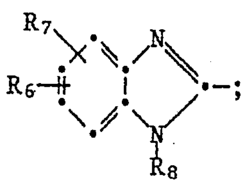 ; oder $Q_{17}$ = 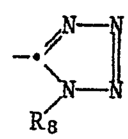

Besonders hervorzuheben sind Verbindungen der Formel I, worin die Gruppe $R_5O$ in para Stellung zur Gruppe Z steht und

Z 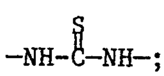 ; 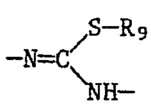 oder $-N=C=N-$;

$R_1$ $C_3$-$C_7$-Alkyl; Cyclopentyl; Cyclohexyl; (Di-cyclopropyl)-methyl; oder Alkoxyalkyl mit 4 C-Atomen;
$R_2$ und $R_3$ unabhängig voneinander $C_1$-$C_3$-Alkyl;
$R_3$ und $R_4$ Wasserstoff;
$R_5$ einen heterozyclischen Rest

$Q_1$ = 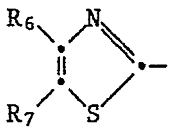 ; $Q_2$ = 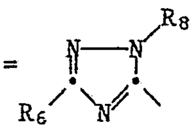 ;

$Q_3$ = 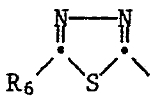 ; $Q_4$ = 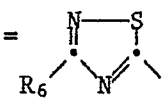 ;

$Q_5$ = 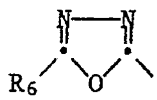 ; $Q_6$ = 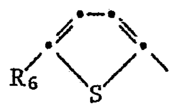 ;

$Q_7$ = 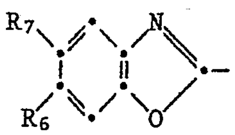 ; $Q_8$ = 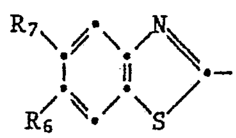 ; oder

$Q_9$ = 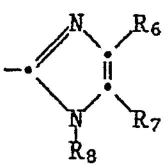 ;

$R_6$ Wasserstoff; Chlor; Brom; $C_1$-$C_4$-Alkyl; $C_1$-$C_3$-Perfluoralkyl; oder Phenyl;
$R_7$ Wasserstoff; Chlor; Brom; oder $C_1$-$C_4$-Alkyl;
$R_8$ Wasserstoff; oder $C_1$-$C_3$-Alkyl;
$R_9$ Methyl; oder Aethyl
bedeuten, sowie deren Salze mit Halogenwasserstoffsäuren.

Insbesondere werden gemäss der Erfindung Verbindungen der Formel I bevorzugt, worin die Gruppe $R_5O$ in para Stellung zur Gruppe Z steht und

Z 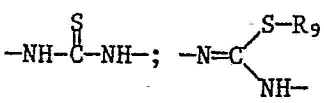 oder —N=C=N—;

$R_1$ tert.-Butyl; tert.-Pentyl; $C_5$-$C_6$-Cycloalkyl; oder 3-Methoxypropyl;
$R_2$ und $R_3$ unabhängig voneinander $C_1$-$C_3$-Alkyl;
$R_4$ Wasserstoff;
$R_5$ einen heterozyclischen Rest

$Q_1$ = 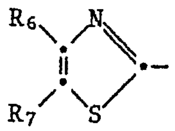 ; $Q_2$ = 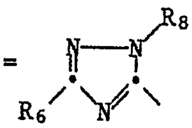 ;

$Q_3$ = 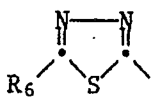 ; $Q_4$ = 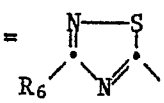 oder

$Q_5$ = 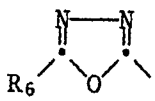 ;

$R_6$ $C_1$-$C_4$-Alkyl; Chlor; Brom; oder $C_1$-$C_3$-Perfluoralkyl;
$R_7$ Wasserstoff; Chlor; Brom; oder $C_1$-$C_4$-Alkyl;
$R_8$ $C_1$-$C_3$-Alkyl;
$R_9$ Methyl; oder Aethyl
bedeuten, sowie deren Salze mit Halogenwasserstoffsäuren.

Die unter die allgemeine Formel I fallenden erfindungsgemässen neuen substituierten Thioharnstoffe (Z = -NH-CS-NH-) haben die Formel Ia

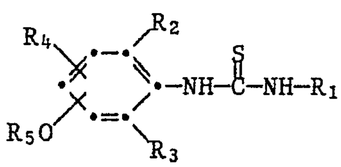 (Ia),

und die neuen substituierten Carbodiimide (Z = -N=C=N-) haben die Formel Ic

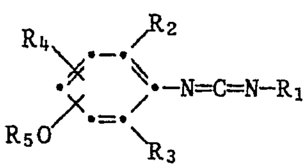 (Ic),

worin die Reste $R_1$ bis $R_5$ wie zuvor definiert sind.

Die erfindungsgemässen neuen substituierten Isothioharnstoffe können in den tautomeren Formeln Ib und Ib"

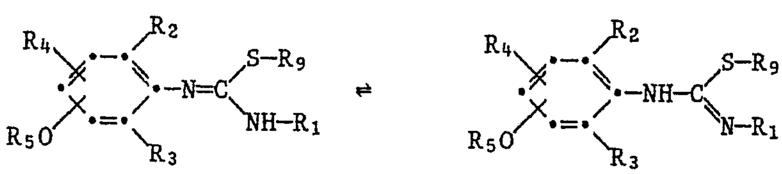

(Ib) (Ib")

vorliegen, wobei $R_1$ bis $R_5$ und $R_9$ die oben angegebenen Bedeutungen haben. Die Erfindung umfasst beide tautomeren Formen als solche, sowie auch Gemische der Tautomeren.

Die Verbindungen der Formel I können auch in Form von Additionssalzen anorganischer oder organischer Säuren vorliegen und erfindungsgemäss in Form ihrer Salze verwendet werden. Unter den Verbindungen der Formel I sind im Sinne der vorliegenden Erfindung demzufolge sowohl die freien Verbindungen der Formel I als auch ihre Säureadditionssalze zu verstehen.

Die Verbindungen der Formel I können nach an sich bekannten Arbeitsweisen in ihre Säuresalze überführt werden. Zur Bildung von Additionssalzen sind anorganische und organische Säuren, beispielsweise Chlorwasserstoffsäure, Bromwasserstoffsäure, Jodwasserstoffsäure, Salpetersäure, Phosphorsäure, Schwefelsäure, Essigsäure, Propionsäure, Buttersäure, Valeriansäure, Oxalsäure, Malonsäure, Bernsteinsäure,

Aepfelsäure, Maleinsäure, Fumarsäure, Milchsäure, Weinsäure, Zitronensäure, Benzoesäure, Phthalsäure, Zimtsäure und Salicylsäure, geeignet.

Unter dem Begriff Alkyl sind im Rahmen der vorliegenden Erfindung geradkettige und verzweigte Alkylreste und je nach Zahl der angegebenen Kohlenstoffatome beispielsweise folgende Gruppen zu verstehen: Methyl, Aethyl, Propyl, Butyl, Pentyl usw. sowie ihre Isomeren, wie z.B. Isopropyl, Isobutyl, tert.-Butyl, Isoamyl, tert. Pentyl usw. Unter Halogen sind Fluor, Chlor, Brom und Jod, vorzugsweise Fluor und Chlor, zu verstehen.

Unter dem Begriff Halogenalkyl im Rahmen der vorliegenden Erfindung sind geradkettige und verzweigte Alkylreste, wie Methyl, Aethyl, n-Propyl, Isopropyl und die isomeren Butylreste zu verstehen, die mit bis zu 9 unterschiedlichen oder gleichartigen Halogenatomen substituiert sind, wobei es sich um perhalogenierte Alkylreste oder auch um solche handeln kann, deren Wasserstoffatome nur teilweise durch Halogen substituiert sind. Als Halogenalkyle sind beispielsweise die Perfluoralkylradikale Trifluormethyl, Pentafluoräthyl oder Heptafluor-n-propyl zu nennen.

Die erfindungsgemässen Verbindungen der Formel I sind neu und können nach an sich bekannten Verfahren hergestellt werden, wie z.B.

a) durch Addition eines Amins der Formel VI an ein Isothiocyanat der Formel II

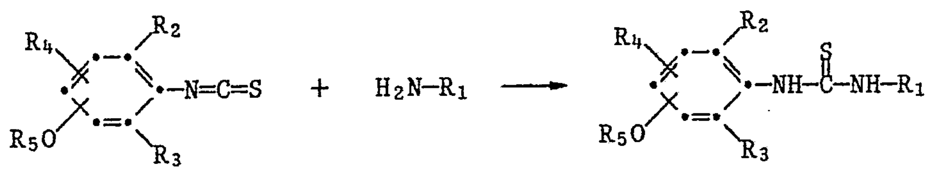

(II) (VI) (Ia)

unter Bildung eines Thioharnstoffs der Formel Ia oder

b) durch Alkylierung eines Thioharnstoffs der Formel Ia mit einer Verbindung der Formel VII, in der

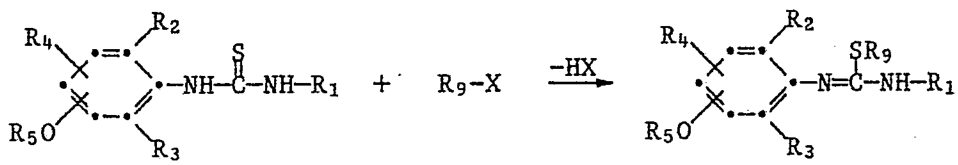


(Ia) (VII) (Ib)

X eine unter den Reaktionsbedingungen abspaltbare Gruppe bedeutet, unter Bildung eines Isothioharnstoffs der Formel Ib. X steht dabei vorzugsweise für ein Halogen, z.B. Chlor, Brom oder Jod, einen gegenenfalls halogenierten oder alkylierten Sulfonsäureester, z.B. ein Mesylat, Brosylat, Tosylat oder ein Dialkylsulfat, z.B. Dimethylsulfat, (vgl. auch J.B.Hendrickson et al., "Organic Chemistry", Mc Graw Hill Book Co, 1970 S.378-382). Ein Isothioharnstoff der Formel Ib kann auch durch Behandlung des unter den Reaktionsbedingungen primär anfallenden Isothiouroniumsalzes mit Basen (z.B. Alkalihydroxiden und -carbonaten) erhalten werden.

c) durch Abspaltung von Schwefelwasserstoff aus einem Thioharnstoff der Formel Ia mittels geeigneter Reagentien, wie etwa 2-Chlor-1-methyl-pyridinium Iodid in Gegenwart einer Base, gemäss nachstehender Gleichung

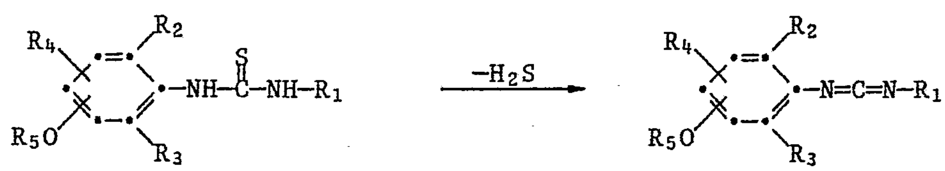


(Ia) (Ic)

unter Bildung eines Carbodiimids der Formel Ic. Die zu den Carbodiimiden der Formel Ic führenden Abspaltungs-Reaktionen können nach literaturbekannten Arbeitsweisen z.B. mit HgO, bestimmten Pyridiniumsalzen, Chloressigsäureestern, Cyanursäurechlorid, p-Toluolsulfochlorid oder bestimmten Phosphorsäureester-Derivaten durchgeführt werden. [vgl. T. Shibanuma, Chemistry Letters (1977), p. 575-6; S. Kim, Tetrahedron Letters (1985), P. 1661-1664; W. Weith, B.6 (1873) 1398; G. Amiard, Bull. Soc. chim. 1956, 1360].

Die obigen Verfahren a), b) und c) werden zweckmässigerweise bei einer Temperatur zwischen 0° C und 150° C, z.B. zwischen 20 und 80° C, bei normalem oder leicht erhöhtem Druck und vorzugsweise in Gegenwart eines gegenüber den Reaktionsteilnehmern inerten Lösungs- oder Verdünnungsmittels durchgeführt. Als

Lösungs-oder Verdünnungsmittel eignen sich z.B. Aether und ätherartige Verbindungen, wie Di-äthyläther, Di-isopropyläther, Dioxan und Tetrahydrofuran; aromatische Kohlenwasserstoffe, wie Benzol, Toluol und Xylole; Ketone, wie Acetone, Methyläthylketon und Cyclohexanon; Dimethylformamid und Acetonitril. Alkohole wie Aethanol, Methanol, Propanol oder Isopropanol, sind nur für die Verfahren a) und b) geeignet.

Die Phenylisothiocyanate der Formel II sind neu und bilden ebenfalls einen Gegenstand der vorliegenden Erfindung. Sie können in an sich bekannter Weise hergestellt werden, z.B. durch Umsetzung eines Anilins der Formel III mit Thiophosgen in Gegenwart einer Base, wie z.B. Calciumcarbonat, (vgl. S. Patai ed. "The Chemistry of Cyanates and their Thioderivatives", part 2, S. 1032 ff, Wiley 1977):

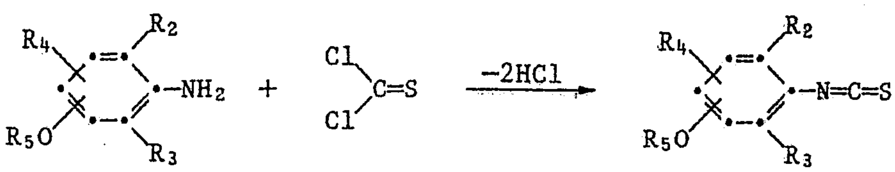


(III) (II)

Auch die Aniline der Formel III sind neuartige Verbindungen. Sie sind beispielsweise dadurch erhältlich, dass man entsprechend substituierte Aminophenole IV in Gegenwart von basischen Substanzen mit Verbindungen der Formel V kondensiert (vgl. J.Am.Soc. Perkin I 1981, 2335; Bull. Soc. Chim. Fr. 1962, 1735; DE-OS 2252070; A. Weissberger et al, "The Chemistry of Heterocyclic Compounds, Vol. 34, I, 565 ff.):

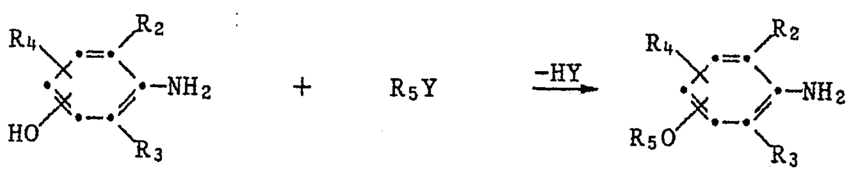


(IV) (V) (III)

Y steht in Formel V für eine unter den Reaktionsbedingungen abspaltbare Gruppe, wie etwa Halogen (vorzugsweise Chlor, Brom oder Jod) oder $C_1$-$C_4$-Alkylsulfonyl (wie etwa Methylsulfonyl). Die Aniline der Formel III sind somit ebenfalls wertvolle Ausgangsverbindungen und Gegenstand dieser Erfindung.

Neben ihrer insektiziden Wirkung sind die Thioharnstoffe der Formel Ia - wie erwähnt - auch wertvolle Ausgangsverbindungen zur Herstellung der Isothioharnstoffe der Formel Ib und der Carbodimide der Formel Ic. Die Verwendung der Thioharnstoffe Ia als Zwischenprodukte zur Herstellung von Ib ind Ic ist somit ebenfalls ein Gegenstand der Erfindung.

Es wurde überraschenderweise gefunden, dass die erfindungsgemässen Verbindungen der Formeln I bei guter Pflanzenverträglichkeit und geringer Warmblütertoxizität ausgezeichnete Wirksamkeit als Schädlingsbekämpfungsmittel aufweisen. Sie eignen sich vor allem zur Bekämpfung von Pflanzen und Tiere befallenden Schädlingen.

Die erfindungsgemässen Verbindungen der Formeln I (bzw. Ia, Ib sowie Ic) weisen eine besonders ausgeprägte Wirkung gegen pflanzenschädigende Akariden (Spinnmilben: z.B. der Familien Tetranychidae, Tarsonemidae, Eriophyidae, Tyroglyphidae und Glycyphagidae) und auch gegen ektoparasitäre Akarinen (Milben und Zecken: z.B. der Familien Ixodidae, Argasidae, Sarcoptidae und Dermanyssidae), welche Nutztiere befallen, auf. Einige der erfindungsgemässen Verbindungen zeichnen sich durch gute akarizid-ovizide Aktivität und Blattpenetrationswirkung aus. Die erfindungsgemässen Verbindungen eignen sich vor allem zur Bekämpfung der folgenden, Obst- und Gemüsekulturen befallenden Milbenspezies: Tetranychus urticae, Tetranychus cinnabarinus, Panonychus ulmi, Bryobia rubrioculus, Panonychus citri, Eriophyes pyri, Eriophyes ribis, Eriophyes vitis, Tarsonemus pallidus, Phyllocoptes vitis und Phyllocoptruta oleivora.

Darüberhinaus wurde festgestellt, dass die Verbindungen der Formel I auch insektizide Eigenschaften besitzen und demzufolge zur Bekämpfung von pflanzenschädigenden bzw. ektoparasitären Insekten, z.B. der Ordnungen Lepidoptera, Coleoptera, Heteroptera, Diptera, Orthoptera und Homoptera, besonders geeignet sind.

Neben ihrer sehr günstigen Wirkung gegenüber Fliegen, wie z.B. Musca domestica, und Mückenlarven eignen sich Verbindungen der Formel I zur Bekämpfung von pflanzenschädigenden Frassinsekten, in Zier- und Nutzpflanzungen, insbesondere in Baumwollkulturen (z.B. Spodoptera littoralis und Heliothis virescens) sowie in Gemüsekulturen (z.B. Leptinotarsa decemlineata und Plutella xylostella).

Die Verbindungen der Formel I eignen sich weiterhin zur Bekämpfung von Ektoparasiten, z.B. Lucilia sericata, an Haus- und Nutztieren, z.B. durch Tier-, Stall- und Weidebehandlung.

Die gute pestizide Wirkung der erfindungsgemässen Verbindungen der Formel I entspricht einer

Abtötungsrate (Mortalität) von mindestens 50-60 % der erwähnten Schädlinge.

Die Wirkung der erfindungsgemässen Verbindungen bzw. der sie enthaltenden Mittel lässt sich durch Zusatz von anderen Insektiziden und/oder Akariziden wesentlich verbreitern und an gegebene Umstände anpassen. Als Zusätze kommen z.B. Vertreter der folgenden Wirkstoffklassen in Betracht: Organische Phosphorverbindungen, Nitrophenole und Derivate, Formamidine, Harnstoffe, Carbamate, Pyrethroide. chlorierte Kohlenwasserstoffe und Bacillus thuringiensis-Präparate.

Die Verbindungen der Formel I werden in unveränderter Form oder vorzugsweise zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren, wie Versprühen. Vernebeln, Verstäuben, Verstreuen oder Giessen, werden ebenso wie die Mittel den angestrebten Zielen und den gegebenen Verhältnissen entsprechend gewählt.

Die Formulierungen, d.h. die die Wirkstoffe der Formel I bzw. Kombinationen dieser Wirkstoffe mit anderen Insektiziden oder Akariziden, und gegebenenfalls einen festen oder flüssigen Zusatzstoff enthaltenden Mittel, Zubereitungen oder Zusammensetzungen, werden in bekannter Weise hergestellt, z.B. durch inniges Vermischen und/oder Vermahlen der Wirkstoffe mit Streckmitteln, wie z.B. mit Lösungsmitteln, festen Trägerstoffen, und gegebenenfalls oberflächenaktiven Verbindungen (Tensiden).

Als Lösungsmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester, wie Dibutyl- oder Dioctylphthalat, aliphatische Kohlenwasserstoffe, wie Cyclohexan, Paraffine, Alkohole und Glykole sowie deren Aether und Ester, wie Aethanol, Aethylenglykol, Aethylenglykolmonomethyl- oder -äthyläther, Ketone, wie Cyclohexanon, stark polare Lösungsmittel, wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl, oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäuren oder hochdisperse saugfähige Polymerisate zugesetzt werden. Als gekörnte, adsorptive Granulatträger kommen poröse Typen, wie z.B. Bimsstein, Ziegelbruch, Sepiolit oder Bentonit, als nicht sorptive Trägermaterialien z.B. Calcit oder Sand in Frage. Darüberhinaus kann eine Vielzahl von granulierten Materialien anorganischer oder organischer Natur, wie insbesondere Dolomit oder zerkleinerte Pflanzenrückstände, verwendet werden.

Als oberflächenaktive Verbindungen kommen je nach Art des zu formulierenden erfindungsgemässen Wirkstoffes oder der Kombinationen dieser Wirkstoffe mit andern Insektiziden oder Akariziden nichtionogene, kation- und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen eignen sich die Alkali-, Erdalkali- oder gegebenenfalls substituierten Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Tallöl gewonnen werden können. Ferner sind als Tenside auch die Fettsäuremethyl-taurin-salze sowie modifizierte und nicht modifizierte Phospholipide zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen im allgemeinen einen Alkylrest mit 8 bis 22 C-Atomen auf, wobei Alkyl auch den Alkylteil von Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches. Hierher gehören auch die Salze der Schwefelsäureester und Sulfonsäuren von Fettalkohol-Aethylenoxid-Addukten. Die sulfonierten Benzimidazolderivate enthalten vorzugsweise 2 Sulfonsäuregruppen und einen Fettsäurerest mit etwa 8-22 C-Atomen. Alkylarylsulfonate sind z.B. die Na-, Ca- oder Triäthanolaminsalze der Dodecylbenzolsulfonsäure, der Dibutylnaphthalinsulfonsäure oder eines Naphthalinsulfonsäure-Formaldehydkondensationsproduktes. Ferner kommen auch entsprechende Phosphate, wie z.B. Salze des Phosphorsäureesters eines p-Nonylphenol-(4-14)-Aethylenoxid-Adduktes, in Frage.

Als nichtionische Tenside kommen in erster Linie Polyglykolätherderivate von aliphatischen oder cycloaliphatischen Alkoholen, gesättigten oder ungesättigten Fettsäuren und Alkylphenolen in Frage, die 3 bis 30 Glykoläthergruppen und 8 bis 20 Kohlenstoffatome im (aliphatischen) Kohlenwasserstoffrest und 6 bis 18 Kohlenstoffatome im Alkylrest der Alkylphenole enthalten können. Weiterhin geeignete nichtionische Tenside sind die wasserlöslichen 20 bis 250 Aethylenglykoläthergruppen und 10 bis 100 Propylenglykoläthergruppen enthaltenden Polyäthylenoxid-Addukte an Polypropylenglykol, Aethylendiaminopolypropylenglykol und Alkylpolypropylenglykol mit 1 bis 10 Kohlenstoffatomen in der Alkylkette. Die genannten Verbindungen enthalten üblicherweise pro Propylenglykol-Einheit 1 bis 5 Aethylenglykoleinheiten.

Als Beispiele nichtionischer Tenside seien Nonylphenolpolyäthoxyäthanole, Ricinusölpolyglykoläther, Polypropylen-Polyäthylenoxid-Addukte, Tributylphenoxypolyäthoxyäthanol, Polyäthylenglykol und Octylphenoxypolyäthoxyäthanol erwähnt. Ferner kommen auch Fettsäureester von Polyoxyäthylensorbitan, wie das Polyoxyäthylensorbitantrioleat in Betracht.

Bei den kationischen Tensiden handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Aethylsulfate vor, z.B. das Stearyltrimethylammoniumchlorid oder das Benzyl-di-(2-chloräthyl)-äthylammoniumbromid.

Die in der Formulierungstechnik gebräuchlichen Tenside sind u.a. in folgenden Publikationen beschrieben: "Mc Cutcheon's Detergents and Emulsifiers Annual" MC Publishing Corp., Ridgewood, New Jersey, 1979; Dr. Helmut Stache "Tensid Taschenbuch", Carl Hanser Verlag München/Wien 1981.

Die pestiziden Zubereitungen enthalten in der Regel 0,1 bis 99 %, insbesondere 0,1 bis 95 %, eines Wirkstoffes der Formel I oder Kombinationen dieses Wirkstoffes mit andern Insektiziden oder Akariziden, 1 bis 99,9 % eines festen oder flüssigen Zusatzstoffes und 0 bis 25 %, insbesondere 0,1 bis 20 %, eines Tensides. Während als Handelsware eher konzentrierte Mittel bevorzugt werden, verwendet der Endverbraucher in der Regel verdünnte Zubereitungen, die wesentlich geringere Wirkstoffkonzentrationen aufweisen, wie z.B. 0,1 bis 1000 ppm.

In den folgenden experimentellen Beispielen sind die (unkorrigierten) Schmelzpunkte in °C angegeben.

Beispiel 1: Herstellung von 2,6-Diemthyl-4-(4-bromthiazol-2-yloxy)anilin

10.0 g 2,4-Dibromthiazol, 5,65 g 3,5-Dimethyl-4-aminophenol, 7,4 g Kaliumcarbonat und 50 ml trockenes Dimethylsulfoxid werden vereinigt und während 23 Stunden auf 90°C gehalten. Man giesst das Reaktionsgemisch auf 300 ml Eiswasser und äthert aus. Die Aetherphasen werden mit 10%iger Natriumhydroxidlösung und Wasser gewaschen, mit Natriumsulfat getrocknet und säulenchromatographisch an Kieselgel mit Hexan/Essigester (1:1) gereinigt.

Man isoliert die Titelverbindung der Formel

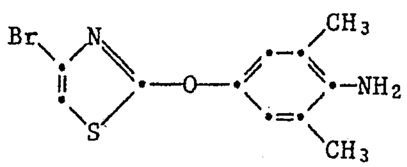

als Kristalle vom Smp. 115-117°C (Verbindung No.1.01).

Entsprechend der vorstehend beschriebenen Arbeitsweise werden die folgenden Aniline der Formel III' hergestellt:

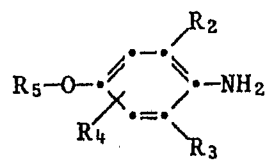

(III')

| Verb. No. | $R_2$ | $R_3$ | $R_4$ | $R_5$ | $R_6$ | $R_7$ | $R_8$ | Phys.Dat. |
|---|---|---|---|---|---|---|---|---|
| 1.01 | $CH_3$ | $CH_3$ | - | $Q_1$ | Br | H | - | F=115-7.5° |
| 1.02 | $C_2H_5$ | $C_2H_5$ | - | $Q_1$ | $CF_3$ | Br | - | F= 72- 4° |
| 1.03 | $CH_3$ | $CH_3$ | - | $Q_1$ | Br | Cl | - | F= 82- 6° |
| 1.04 | $CH_3$ | $CH_3$ | - | $Q_1$ | Br | Br | - | F= 90- 2° |
| 1.05 | $CH_3$ | $CH_3$ | - | $Q_1$ | Cl | Br | - | F= 86- 8° |
| 1.06 | $CH_3$ | $CH_3$ | - | $Q_1$ | Cl | Cl | - | F= 74.5-6° |
| 1.07 | $CH_3$ | $CH_3$ | - | $Q_1$ | Cl | H | - | F=120° |
| 1.08 | $CH_3$ | $CH_3$ | - | $Q_1$ | $CF_3$ | H | - | F=109- 10° |
| 1.09 | $CH_3$ | $CH_3$ | - | $Q_1$ | $CF_3$ | Br | - | F= 86- 87° |
| 1.10 | $C_2H_5$ | $C_2H_5$ | - | $Q_1$ | Cl | Cl | - | F= 58- 60° |
| 1.11 | $C_2H_5$ | $C_2H_5$ | - | $Q_1$ | Cl | Br | - | F= 55- 56° |
| 1.12 | $CH_3$ | $CH_3$ | - | $Q_2$ | $CF_3$ | - | $CH_3$ | F=155-156° |

| Verb. No. | $R_2$ | $R_3$ | $R_4$ | $R_5$ | $R_6$ | $R_7$ | $R_8$ | Phys.Dat. |
|---|---|---|---|---|---|---|---|---|
| 1.13 | $CH_3$ | $CH_3$ | - | $Q_2$ | $CF_3$ | - | $C_3H_7(i)$ | F=153-155° |
| 1.14 | $CH_3$ | $CH_3$ | - | $Q_2$ | $C_2F_5$ | - | $CH_3$ | F=125-128° |
| 1.15 | $CH_3$ | $CH_3$ | - | $Q_2$ | $C_3F_7(n)$ | - | $CH_3$ | F=130-133° |
| 1.16 | $CH_3$ | $CH_3$ | - | $Q_2$ | $C_2F_5$ | - | $C_3H_7(i)$ | F=146-149° |
| 1.17 | $CH_3$ | $CH_3$ | - | $Q_2$ | $C_3F_7(n)$ | - | $C_3H_7(i)$ | F=133-135° |
| 1.18 | $C_2H_5$ | $C_2H_5$ | - | $Q_2$ | $CF_3$ | - | $C_3H_7(i)$ | F=128-130° |
| 1.19 | $C_2H_5$ | $C_2H_5$ | - | $Q_2$ | $C_2F_5$ | - | $CH_3$ | F=121-122° |
| 1.20 | $C_2H_5$ | $C_2H_5$ | - | $Q_2$ | $C_2F_5$ | - | $C_3H_7(i)$ | F=106-108° |
| 1.21 | $C_2H_5$ | $C_2H_5$ | - | $Q_2$ | $C_3F_7(n)$ | - | $CH_3$ | F=119-120° |
| 1.22 | $C_2H_5$ | $C_2H_5$ | - | $Q_2$ | $C_3F_7(n)$ | - | $C_3H_7(i)$ | F=113-114° |
| 1.23 | $C_2H_5$ | $C_2H_5$ | - | $Q_2$ | $CF_3$ | - | $CH_3$ | F=130-131° |
| 1.24 | $C_2H_5$ | $C_2H_5$ | - | $Q_3$ | $C_4H_9(t)$ | - | - | F=128-130° |
| 1.25 | $CH_3$ | $CH_3$ | - | $Q_3$ | $CF_3$ | - | - | F= 80- 84° |
| 1.26 | $CH_3$ | $CH_3$ | - | $Q_4$ | $CH_3$ | - | - | F=128-130° |

| Verb. No. | $R_2$ | $R_3$ | $R_4$ | $R_5$ | $R_6$ | $R_7$ | $R_8$ | Phys.Dat. |
|---|---|---|---|---|---|---|---|---|
| 1.27 | $CH_3$ | $CH_3$ | - | $Q_9$ | Cl | Cl | $CH_3$ | F=176,5-177° |
| 1.28 | $C_2H_5$ | $C_2H_5$ | - | $Q_9$ | Cl | Cl | $CH_3$ | F=120,5-121,5° |
| 1.29 | $C_3H_7(i)$ | $C_3H_7(i)$ | - | $Q_4$ | $CH_3$ | - | - | Feststoff |
| 1.30 | $CH_3$ | $CH_3$ | - | $Q_1$ | Cl | $CH_3$ | - | $n_D^{23}$ = 1,6075 |
| 1.31 | $C_2H_5$ | $C_2H_5$ | - | $Q_1$ | Cl | $CH_3$ | - | $n_D^{25}$ = 1,5930 |
| 1.32 | $C_3H_7(i)$ | $C_3H_7(i)$ | - | $Q_1$ | Cl | Cl | - | dunkles Oel*) |
| 1.33 | $CH_3$ | $CH_3$ | - | $Q_8$ | Cl | Cl | - | F = 166-170° |

*) nmr ($CDCl_3$):
$\delta$ = 1,25 (d,12)
2,88 (septett, 2)
3,95 (breit, $NH_2$)
6,85 (s,2)

In analoger Weise sind auch die folgenden Aniline der Formel III' herstellbar:

| $R_2$ | $R_3$ | $R_4$ | $R_5$ | $R_6$ | $R_7$ | $R_8$ |
|---|---|---|---|---|---|---|
| $C_3H_7$(i) | $C_3H_7$(i) | - | $Q_7$ | H | H | - |
| $CH_3$ | $CH_3$ | - | $Q_7$ | 6-Cl | H | - |
| $C_2H_5$ | $C_2H_5$ | - | $Q_7$ | 6-Cl | H | - |
| $C_3H_7$(i) | $C_3H_7$(i) | - | $Q_7$ | 6-Cl | H | - |
| $CH_3$ | $CH_3$ | - | $Q_8$ | 6-Cl | H | - |
| $C_2H_5$ | $C_2H_5$ | - | $Q_8$ | 6-Cl | H | - |
| $C_3H_7$(i) | $C_3H_7$(i) | - | $Q_8$ | 6-Cl | H | - |
| $CH_3$ | $CH_3$ | - | $Q_4$ | $CF_3$ | - | - |
| $C_2H_5$ | $C_2H_5$ | - | $Q_4$ | $CF_3$ | - | - |
| $C_3H_7$(i) | $C_3H_7$(i) | - | $Q_4$ | $CF_3$ | - | - |
| $C_3H_7$(i) | $C_3H_7$(i) | - | $Q_9$ | Cl | Cl | $CH_3$ |
| $CH_3$ | $CH_3$ | - | $Q_4$ | $CF_3$ | - | - |
| $C_2H_5$ | $C_2H_5$ | - | $Q_4$ | $CF_3$ | - | - |
| $C_3H_7$(i) | $C_3H_7$(i) | - | $Q_4$ | $CF_3$ | - | - |
| $CH_3$ | $CH_3$ | - | $Q_9$ | Cl | Cl | H |
| $C_2H_5$ | $C_2H_5$ | - | $Q_9$ | Cl | Cl | H |
| $C_3H_7$(i) | $C_3H_7$(i) | - | $Q_9$ | Cl | Cl | H |
| $CH_3$ | $CH_3$ | - | $Q_9$ | Br | Br | H |
| $C_2H_5$ | $C_2H_5$ | - | $Q_9$ | Br | Br | H |
| $C_3H_7$(i) | $C_3H_7$(i) | - | $Q_9$ | Br | Br | H |
| $CH_3$ | $CH_3$ | - | $Q_6$ | H | H | - |
| $C_2H_5$ | $C_2H_5$ | - | $Q_6$ | H | H | - |
| $C_2H_5$ | $CH_3$ | - | $Q_6$ | H | H | - |

| $R_2$ | $R_3$ | $R_4$ | $R_5$ | $R_6$ | $R_7$ | $R_8$ |
|---|---|---|---|---|---|---|
| $C_3H_7$(i) | $C_3H_7$(i) | - | $Q_6$ | H | H | - |
| $CH_3$ | $CH_3$ | - | $Q_4$ | $C_6H_5$ | - | - |
| $C_2H_5$ | $C_2H_5$ | - | $Q_4$ | $C_6H_5$ | - | - |
| $C_3H_7$(i) | $C_3H_7$(i) | - | $Q_4$ | $C_6H_5$ | - | - |
| $CH_3$ | $CH_3$ | - | $Q_7$ | H | - | - |
| $C_2H_5$ | $C_2H_5$ | - | $Q_7$ | H | - | - |
| $C_2H_5$ | $C_2H_5$ | - | $Q_1$ | $CF_3$ | Br | - |
| $C_2H_5$ | $C_2H_5$ | - | $Q_4$ | $CH_3$ | - | - |
| $CH_3$ | $CH_3$ | - | $Q_3$ | $C_4H_9$(t) | - | - |
| $CH_3$ | $CH_3$ | - | $Q_5$ | $C_4H_9$(t) | - | - |
| $C_3H_7$(i) | $C_3H_7$(i) | - | $Q_1$ | Cl | $CH_3$ | - |
| $CH_3$ | $CH_3$ | - | $Q_1$ | $CH_3$ | Cl | - |
| $C_2H_5$ | $C_2H_5$ | - | $Q_1$ | $CH_3$ | Cl | - |
| $C_3H_7$(i) | $C_3H_7$(i) | - | $Q_1$ | $CH_3$ | Cl | - |
| $CH_3$ | $CH_3$ | - | $Q_1$ | $CH_3$ | Br | - |
| $C_2H_5$ | $C_2H_5$ | - | $Q_1$ | $CH_3$ | Br | - |
| $C_3H_7$(i) | $C_3H_7$(i) | - | $Q_1$ | $CH_3$ | Br | - |
| $CH_3$ | $CH_3$ | - | $Q_1$ | $CH_3$ | $CH_3$ | - |
| $C_2H_5$ | $C_2H_5$ | - | $Q_1$ | $CH_3$ | $CH_3$ | - |
| $C_3H_7$(i) | $C_3H_7$(i) | - | $Q_1$ | $CH_3$ | $CH_3$ | - |
| $CH_3$ | $CH_3$ | - | $Q_4$ | $C_2H_5$ | - | - |
| $C_2H_5$ | $C_2H_5$ | - | $Q_4$ | $C_2H_5$ | - | - |
| $C_3H_7$(i) | $C_3H_7$(i) | - | $Q_4$ | $C_2H_5$ | - | - |
| $CH_3$ | $CH_3$ | - | $Q_4$ | $C_3H_7$(i) | - | - |
| $C_2H_5$ | $C_2H_5$ | - | $Q_4$ | $C_3H_7$(i) | - | - |
| $C_3H_7$(i) | $C_3H_7$(i) | - | $Q_4$ | $C_3H_7$(i) | - | - |

In analoger Weise wie in Beispiel 1 angegeben werden auch die folgenden Aniline der Formel III hergestellt:

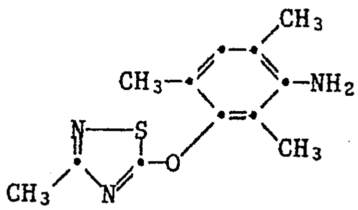

F = 96-98°C (Verbindung Nr. 1.34)

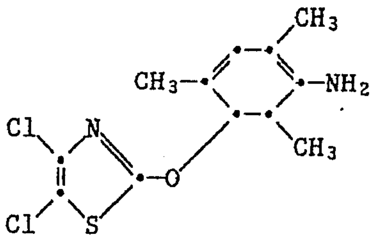

F = 89-90°C (Verbindung Nr. 1.35)

Beispiel 2: Herstellung von 2,6-Dimethyl-4(4-brom-1,3-thiazol-2-yloxy)phenyl isothiocyanat

Zu einer Mischung von 15 ml Wasser, 30 ml Dichlormethan, 2,1 g Thiophosgen und 3,3 g Calciumcarbonat werden unter Rühren und Eiskühlung 4,5 g 2,6-Dimethyl-4-(4-brom-1,3-thiazol-2-yloxy)-anilin zugetropft. Danach wird für 2 Stunden zum Sieden erhitzt. Nach dem Erkalten wird das Reaktionsgemisch filtriert, die organische Phase abgetrennt, die wässrige Phase mit Dichlormethan gewaschen und die vereinigten organischen Phasen über Natriumsulfat getrocknet. Nach Abdestillieren des Lösungsmittels verbleiben hellgelbe Kristalle die aus Hexan umkristallisiert werden. Man isoliert die Titelverbindung der Formel

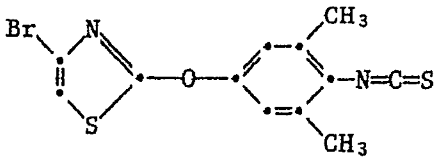

als gelbe Kristalle von Smp. 91.5-93°C (Verbindung No.2.01).

Entsprechend den vorstehend beschriebenen Arbeitsweise werden die folgenden Isothiocyanate der Formel II'

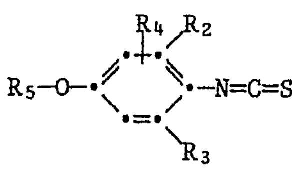

(II')

hergestellt:

| Verb.No. | $R_2$ | $R_3$ | $R_4$ | $R_5$ | $R_6$ | $R_7$ | $R_8$ | Phys.Dat. |
|---|---|---|---|---|---|---|---|---|
| 2.01 | $CH_3$ | $CH_3$ | – | $Q_1$ | Br | H | – | F=91,5–93° |
| 2.02 | $C_2H_5$ | $C_2H_5$ | – | $Q_1$ | $CF_3$ | Br | – | F=82–86° |
| 2.03 | $CH_3$ | $CH_3$ | – | $Q_1$ | Br | Cl | – | F= 76–77,4° |
| 2.04 | $CH_3$ | $CH_3$ | – | $Q_1$ | Br | Br | – | F= 90– 2° |
| 2.05 | $CH_3$ | $CH_3$ | – | $Q_1$ | Cl | Br | – | F=94,5–4° |
| 2.06 | $CH_3$ | $CH_3$ | – | $Q_1$ | Cl | Cl | – | F=73,5–4° |
| 2.07 | $CH_3$ | $CH_3$ | – | $Q_1$ | Cl | H | – | F= 87– 91° |
| 2.08 | $CH_3$ | $CH_3$ | – | $Q_1$ | $CF_3$ | H | – | F=102– 3° |
| 2.09 | $CH_3$ | $CH_3$ | – | $Q_1$ | $CF_3$ | Br | – | F=113– 15° |
| 2.10 | $C_2H_5$ | $C_2H_5$ | – | $Q_1$ | Cl | Cl | – | $n_D^{22}$ =1,6410 |
| 2.11 | $C_2H_5$ | $C_2H_5$ | – | $Q_1$ | Cl | Br | – | F=47,5– 9° |
| 2.12 | $CH_3$ | $CH_3$ | – | $Q_2$ | $CF_3$ | Br | $CH_3$ | Oel |
| 2.13 | $CH_3$ | $CH_3$ | – | $Q_2$ | $CF_3$ | – | $C_3H_7(i)$ | F= 93– 95 |
| 2.14 | $CH_3$ | $CH_3$ | – | $Q_2$ | $C_2F_5$ | – | $CH_3$ | F= 85– 86 |
| 2.15 | $CH_3$ | $CH_3$ | – | $Q_2$ | $C_3F_7(n)$ | – | $CH_3$ | F= 83– 84 |

| Verb.No. | $R_2$ | $R_3$ | $R_4$ | $R_5$ | $R_6$ | $R_7$ | $R_8$ | Phys.Dat. |
|---|---|---|---|---|---|---|---|---|
| 2.16 | $CH_3$ | $CH_3$ | – | $Q_2$ | $C_2F_5$ | – | $C_3H_7$(i) | F= 72- 74 |
| 2.17 | $CH_3$ | $CH_3$ | – | $Q_2$ | $C_3F_7$(n) | – | $C_3H_7$(i) | F=101-102 |
| 2.18 | $C_2H_5$ | $C_2H_5$ | – | $Q_2$ | $C_3H_7$(i) | – | $C_3H_7$(i) | F= 83- 84 |
| 2.19 | $C_2H_5$ | $C_2H_5$ | – | $Q_2$ | $C_2F_5$ | – | $CH_3$ | F= 68- 70 |
| 2.20 | $C_2H_5$ | $C_2H_5$ | – | $Q_2$ | $C_2F_5$ | – | $C_3H_7$(i) | F= 42- 43 |
| 2.21 | $C_2H_5$ | $C_2H_5$ | – | $Q_2$ | $C_3F_7$(n) | – | $CH_3$ | F= 55- 56 |
| 2.22 | $C_2H_5$ | $C_2H_5$ | – | $Q_2$ | $C_3F_7$(n) | – | $C_3H_7$(i) | Oel |
| 2.23 | $C_2H_5$ | $C_2H_5$ | – | $Q_2$ | $CF_3$ | – | $CH_3$ | F=111-112 |
| 2.24 | $C_2H_5$ | $C_2H_5$ | – | $Q_3$ | $C_4H_9$(t) | – | – | F= 97- 98 |
| 2.25 | $CH_3$ | $CH_3$ | – | $Q_3$ | $CF_3$ | – | – | F= 41- 44 |
| 2.26 | $CH_3$ | $CH_3$ | – | $Q_4$ | $CH_3$ | – | – | Oel |
| 2.27 | $CH_3$ | $CH_3$ | – | $Q_9$ | Cl | Cl | $CH_3$ | F=108,5-109 |
| 2.28 | $C_2H_5$ | $C_2H_5$ | – | $Q_9$ | Cl | Cl | $CH_3$ | F=110-111 |
| 2.29 | $C_3H_7$(i) | $C_3H_7$(i) | – | $Q_4$ | $CH_3$ | – | – | Oel |
| 2.30 | $CH_3$ | $CH_3$ | – | $Q_1$ | Cl | $CH_3$ | – | F=112,5-113 |
| 2.31 | $C_2H_5$ | $C_2H_5$ | – | $Q_1$ | Cl | $CH_3$ | – | $n_D^{26}$ = 1,6300 |

| Verb.No. | $R_2$ | $R_3$ | $R_4$ | $R_5$ | $R_6$ | $R_7$ | $R_8$ | Phys.Dat. |
|---|---|---|---|---|---|---|---|---|
| 2.32 | $C_3H_7(i)$ | $C_3H_7(i)$ | - | $Q_1$ | Cl | Cl | - | Oel |
| 2.33 | $CH_3$ | $CH_3$ | - | $Q_8$ | Cl | Cl | - | F=117-118,5° |

Wie vorstehend beschrieben, sind auch die folgenden Isothiocyanate der Formel II' darstellbar:

| $R_2$ | $R_3$ | $R_4$ | $R_5$ | $R_6$ | $R_7$ | $R_8$ |
|---|---|---|---|---|---|---|
| $CH_3$ | $CH_3$ | - | $Q_3$ | $C_4H_9$(t) | - | - |
| $C_3H_7$(n) | $C_3H_7$(n) | - | $Q_4$ | $CH_3$ | - | $CH_3$ |
| $CH_3$ | $CH_3$ | - | $Q_5$ | $C_4H_9$(t) | - | - |
| $C_2H_5$ | $C_2H_5$ | - | $Q_1$ | $CF_3$ | Br | - |
| $C_2H_5$ | $CH_3$ | - | $Q_1$ | $CF_3$ | Br | - |
| $CH_3$ | $CH_3$ | - | $Q_6$ | H | H | - |
| $C_2H_5$ | $CH_3$ | - | $Q_6$ | H | H | - |
| $C_2H_5$ | $C_2H_5$ | - | $Q_6$ | H | H | - |
| $C_3H_7$(i) | $C_3H_7$(i) | - | $Q_6$ | H | H | - |
| $CH_3$ | $CH_3$ | - | $Q_4$ | $C_6H_5$ | - | - |
| $C_2H_5$ | $C_2H_5$ | - | $Q_4$ | $C_6H_5$ | - | - |
| $C_3H_7$(i) | $C_3H_7$(i) | - | $Q_4$ | $C_6H_5$ | - | - |
| $CH_3$ | $CH_3$ | - | $Q_7$ | H | - | - |
| $C_2H_5$ | $C_2H_5$ | - | $Q_7$ | H | - | - |
| $C_3H_7$(i) | $C_3H_7$(i) | - | $Q_7$ | H | - | - |
| $CH_3$ | $CH_3$ | - | $Q_7$ | 6-Cl | - | - |
| $C_2H_5$ | $C_2H_5$ | - | $Q_7$ | 6-Cl | - | - |
| $C_3H_7$(i) | $C_3H_7$(i) | - | $Q_7$ | 6-Cl | - | - |
| $CH_3$ | $CH_3$ | - | $Q_8$ | 6-Cl | H | - |
| $C_2H_5$ | $C_2H_5$ | - | $Q_8$ | 6-Cl | H | - |
| $C_3H_7$(i) | $C_3H_7$(i) | - | $Q_8$ | 6-Cl | H | - |
| $C_2H_5$ | $C_2H_5$ | - | $Q_4$ | $CH_3$ | - | - |

| $R_2$ | $R_3$ | $R_4$ | $R_5$ | $R_6$ | $R_7$ | $R_8$ |
|---|---|---|---|---|---|---|
| $CH_3$ | $CH_3$ | – | $Q_4$ | $CF_3$ | – | – |
| $C_2H_5$ | $C_2H_5$ | – | $Q_4$ | $CF_3$ | – | – |
| $C_3H_7(i)$ | $C_3H_7(i)$ | – | $Q_4$ | $CF_3$ | – | – |
| $C_3H_7(i)$ | $C_3H_7(i)$ | – | $Q_9$ | Cl | Cl | $CH_3$ |
| $CH_3$ | $CH_3$ | – | $Q_4$ | $CF_3$ | – | – |
| $C_2H_5$ | $C_2H_5$ | – | $Q_4$ | $CF_3$ | – | – |
| $C_3H_7(i)$ | $C_3H_7(i)$ | – | $Q_4$ | $CF_3$ | – | – |
| $CH_3$ | $CH_3$ | – | $Q_9$ | Cl | Cl | H |
| $C_2H_5$ | $C_2H_5$ | – | $Q_9$ | Cl | Cl | H |
| $C_3H_7(i)$ | $C_3H_7(i)$ | – | $Q_9$ | Cl | Cl | H |
| $CH_3$ | $CH_3$ | – | $Q_9$ | Br | Br | H |
| $C_2H_5$ | $C_2H_5$ | – | $Q_9$ | Br | Br | H |
| $C_3H_7(i)$ | $C_3H_7(i)$ | – | $Q_9$ | Br | Br | H |
| $C_3H_7(i)$ | $C_3H_7(i)$ | – | $Q_1$ | Cl | $CH_3$ | – |
| $CH_3$ | $CH_3$ | – | $Q_1$ | $CH_3$ | Cl | – |
| $C_2H_5$ | $C_2H_5$ | – | $Q_1$ | $CH_3$ | Cl | – |
| $C_3H_7(i)$ | $C_3H_7(i)$ | – | $Q_1$ | $CH_3$ | Cl | – |
| $CH_3$ | $CH_3$ | – | $Q_1$ | $CH_3$ | Br | – |
| $C_2H_5$ | $C_2H_5$ | – | $Q_1$ | $CH_3$ | Br | – |
| $C_3H_7(i)$ | $C_3H_7(i)$ | – | $Q_1$ | $CH_3$ | Br | – |
| $CH_3$ | $CH_3$ | – | $Q_1$ | $CH_3$ | $CH_3$ | – |
| $C_2H_5$ | $C_2H_5$ | – | $Q_1$ | $CH_3$ | $CH_3$ | – |
| $C_3H_7(i)$ | $C_3H_7(i)$ | – | $Q_1$ | $CH_3$ | $CH_3$ | – |
| $CH_3$ | $CH_3$ | – | $Q_4$ | $C_2H_5$ | – | – |

| $R_2$ | $R_3$ | $R_4$ | $R_5$ | $R_6$ | $R_7$ | $R_8$ |
|---|---|---|---|---|---|---|
| $C_2H_5$ | $C_2H_5$ | - | $Q_4$ | $C_2H_5$ | - | - |
| $C_3H_7(i)$ | $C_3H_7(i)$ | - | $Q_4$ | $C_2H_5$ | - | - |
| $CH_3$ | $CH_3$ | - | $Q_4$ | $C_3H_7(i)$ | - | - |
| $C_2H_5$ | $C_2H_5$ | - | $Q_4$ | $C_3H_7(i)$ | - | - |
| $C_3H_7(i)$ | $C_3H_7(i)$ | - | $Q_4$ | $C_3H_7(i)$ | - | - |

In analoger Weise wie in Beispiel 2 angegeben werden auch die folgenden Isothiocyanate der Formel II hergestellt:

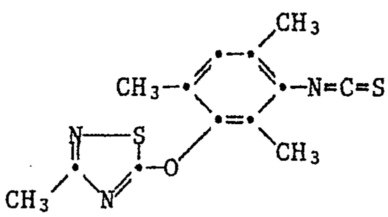

F = 104-106°C
(Verbindung
Nr. 2.34)

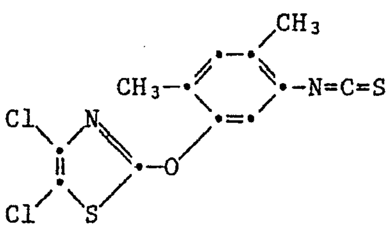

F = 100-101°C
(Verbindung
Nr. 2.35)

Beispiel 3: Herstellung von N-[2,6-Dimethyl-4-(4-bromthiazol-2-yloxy)-phenyl]-N'-tert.butyl-thioharnstoff

2,1 g 2,6-Dimethyl-4-(4-bromthiazol-2-yloxy)-phenylisothiocyanat und 0,7 g tert.Butylamin werden in 20 ml Tetrahydrofuran während 6 Stunden auf 40°C erwärmt. Man giesst den Ansatz auf 200 ml Eiswasser und filtriert den Niederschlag ab. Nach Umkristallisation desselben aus Methanol erhält man die Titelverbindung der Formel

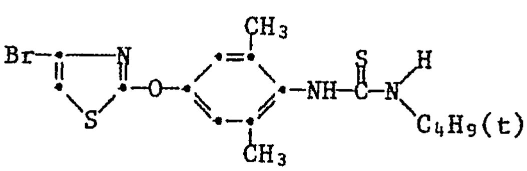

in kristalliner Form mit einem Smp. von 143-144°C (Verbindung Nr.3.01).

Entsprechend den vorstehend beschriebenen Arbeitsweisen werden die folgenden Thioharnstoffe der Formel Ia' hergestellt:

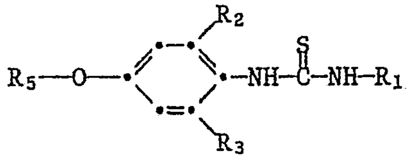 (Ia')

| Verb.No | $R_1$ | $R_2$ | $R_3$ | $R_5$ | $R_6$ | $R_7$ | $R_8$ | Phys. Dat. |
|---|---|---|---|---|---|---|---|---|
| 3.01 | $C_4H_9(t)$ | $CH_3$ | $CH_3$ | $Q_1$ | Br | H | – | F=143-144° |
| 3.02 | $C_4H_9(t)$ | $CH_3$ | $CH_3$ | $Q_1$ | $CF_3$ | H | – | F=168-169° |
| 3.03 | $C_4H_9(t)$ | $CH_3$ | $CH_3$ | $Q_1$ | $CF_3$ | Br | – | F=152-154° |
| 3.04 | $(CH_2)_3OCH_3$ | $CH_3$ | $CH_3$ | $Q_1$ | $CF_3$ | Br | – | F=132-133° |
| 3.05 | 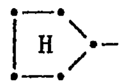 | $CH_3$ | $CH_3$ | $Q_1$ | $CF_3$ | H | – | F=131-132° |
| 3.06 | 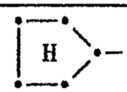 | $CH_3$ | $CH_3$ | $Q_1$ | $CF_3$ | Br | – | F=144-146° |
| 3.07 | 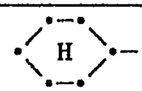 | $CH_3$ | $CH_3$ | $Q_1$ | $CF_3$ | H | – | F=165-166° |
| 3.08 | $C_5H_{11}(t)$ | $CH_3$ | $CH_3$ | $Q_1$ | $CF_3$ | Br | – | F=110-114° |
| 3.09 | $C_4H_9(t)$ | $CH_3$ | $CH_3$ | $Q_1$ | Cl | H | – | F=130-132° |
| 3.10 | $C_4H_9(t)$ | $CH_3$ | $CH_3$ | $Q_1$ | Cl | Cl | – | F=120-123° |
| 3.11 | $C_4H_9(t)$ | $CH_3$ | $CH_3$ | $Q_1$ | Cl | Br | – | F=157-159° |
| 3.12 | $C_4H_9(t)$ | $CH_3$ | $CH_3$ | $Q_1$ | Br | Br | – | F=163-165° |
| 3.13 | $C_5H_{11}(t)$ | $CH_3$ | $CH_3$ | $Q_1$ | Br | H | – | F=144-146° |
| 3.14 | $C_4H_9(t)$ | $C_2H_5$ | $C_2H_5$ | $Q_1$ | $CF_3$ | Br | – | F=150-151,5° |

| Verb.No | $R_1$ | $R_2$ | $R_3$ | $R_5$ | $R_6$ | $R_7$ | $R_8$ | Phys. Dat. |
|---|---|---|---|---|---|---|---|---|
| 3.15 | $C_4H_9$(t) | $CH_3$ | $CH_3$ | $Q_1$ | Br | Cl | - | F=142-144° |
| 3.16 | $C_4H_9$(t) | $C_2H_5$ | $C_2H_5$ | $Q_1$ | Cl | Cl | - | F=114-116,5° |
| 3.17 | $C_4H_9$(t) | $C_2H_5$ | $C_2H_5$ | $Q_1$ | Cl | Br | - | F=139-141° |
| 3.18 | $C_4H_9$(t) | $CH_3$ | $CH_3$ | $Q_2$ | $CF_3$ | - | $CH_3$ | F=169-171° |
| 3.19 | $C_4H_9$(t) | $CH_3$ | $CH_3$ | $Q_2$ | $CF_3$ | - | $C_3H_7$(i) | F=184-185° |
| 3.20 | $C_4H_9$(t) | $CH_3$ | $CH_3$ | $Q_2$ | $C_2F_5$ | - | $CH_3$ | F=135-136° |
| 3.21 | $C_4H_9$(t) | $CH_3$ | $CH_3$ | $Q_2$ | $C_3F_7$(n) | - | $CH_3$ | F=116-118° |
| 3.22 | $C_4H_9$(t) | $CH_3$ | $CH_3$ | $Q_3$ | $C_4H_9$(t) | - | - | F=158-160° |
| 3.23 | $C_4H_9$(t) | $C_2H_5$ | $C_2H_5$ | $Q_9$ | Cl | Cl | $CH_3$ | F=146-147,5° |
| 3.24 | $C_4H_9$(t) | $CH_3$ | $CH_3$ | $Q_9$ | Cl | Cl | $CH_3$ | F=162-163° |
| 3.25 | 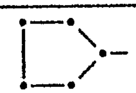 | $C_2H_5$ | $C_2H_5$ | $Q_1$ | Cl | Cl | - | F=125-127° |
| 3.26 | 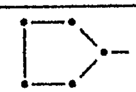 | $C_2H_5$ | $C_2H_5$ | $Q_1$ | Cl | Br | - | F=157-159° |
| 3.27 | $C_4H_9$(t) | $CH_3$ | $CH_3$ | $Q_5$ | $C_4H_9$(t) | - | - | F=168-170° |
| 3.28 | $C_4H_9$(t) | $CH_3$ | $CH_3$ | $Q_2$ | $C_2F_5$ | - | $C_3H_7$(i) | F=151-152° |
| 3.29 | $C_4H_9$(t) | $CH_3$ | $CH_3$ | $Q_2$ | $C_3F_7$(n) | - | $C_3H_7$(i) | F=114-116° |

| Verb.No | $R_1$ | $R_2$ | $R_3$ | $R_5$ | $R_6$ | $R_7$ | $R_8$ | Phys. Dat. |
|---|---|---|---|---|---|---|---|---|
| 3.30 | $C_4H_9$(t) | $C_2H_5$ | $C_2H_5$ | $Q_2$ | $CF_3$ | - | $C_3H_7$(i) | F=163-165° |
| 3.31 | $C_4H_9$(t) | $C_2H_5$ | $C_2H_5$ | $Q_2$ | $C_2F_5$ | - | $CH_3$ | F=114-115° |
| 3.32 | $C_4H_9$(t) | $C_2H_5$ | $C_2H_5$ | $Q_2$ | $C_2F_5$ | - | $C_3H_7$(i) | F=155-156° |
| 3.33 | $C_4H_9$(t) | $C_2H_5$ | $C_2H_5$ | $Q_2$ | $C_3F_7$(n) | - | $CH_3$ | F=105-106° |
| 3.34 | $C_4H_9$(t) | $C_2H_5$ | $C_2H_5$ | $Q_2$ | $C_3F_7$(n) | - | $C_3H_7$(i) | F=130-131° |
| 3.35 | $C_4H_9$(t) | $C_2H_5$ | $C_2H_5$ | $Q_2$ | $CF_3$ | - | $CH_3$ | F=145-146° |
| 3.36 | $C_4H_9$(t) | $C_2H_5$ | $C_2H_5$ | $Q_3$ | $C_4H_9$(t) | - | - | F=147-149° |
| 3.37 | $C_4H_9$(t) | $CH_3$ | $CH_3$ | $Q_3$ | $CF_3$ | - | - | F=118-120° |
| 3.38 | $C_3H_7$(i) | $CH_3$ | $CH_3$ | $Q_3$ | $CF_3$ | - | - | F=103-106° |
| 3.39 | $C_4H_9$(t) | $CH_3$ | $CH_3$ | $Q_4$ | $CH_3$ | - | - | F=152-154° |
| 3.40 | $C_3H_7$(i) | $CH_3$ | $CH_3$ | $Q_4$ | $CH_3$ | - | - | F=163-165° |
| 3.41 | $C_4H_9$(t) | $CH_3$ | $CH_3$ | $Q_9$ | Cl | Cl | $CH_3$ | F=162-163° |
| 3.42 | $C_4H_9$(t) | $C_2H_5$ | $C_2H_5$ | $Q_9$ | Cl | Cl | $CH_3$ | F=146-147,5° |
| 3.43 | $C_4H_9$(t) | $C_3H_7$(i) | $C_3H_7$(i) | $Q_4$ | $CH_3$ | - | - | F=128-130° |
| 3.44 | $C_4H_9$(t) | $CH_3$ | $CH_3$ | $Q_1$ | Cl | $CH_3$ | - | F=145-147° |
| 3.45 | $C_4H_9$(t) | $C_2H_5$ | $C_2H_5$ | $Q_1$ | Cl | $CH_3$ | - | F=113,5-115,5° |

| Verb.No | $R_1$ | $R_2$ | $R_3$ | $R_5$ | $R_6$ | $R_7$ | $R_8$ | Phys. Dat. |
|---|---|---|---|---|---|---|---|---|
| 3.46 | $C_4H_9(t)$ | $C_3H_7(i)$ | $C_3H_7(i)$ | $Q_1$ | Cl | Cl | - | F=120-124° |
| 3.47 | 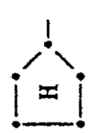 | $C_3H_7(i)$ | $C_3H_7(i)$ | $Q_1$ | Cl | Cl | - | F=146-148,5° |
| 3.48 | $C_4H_9(t)$ | $CH_3$ | $CH_3$ | $Q_8$ | 6-Cl | 5-Cl | - | F=174-176° |
| 3.49 | $C_3H_7(i)$ | $CH_3$ | $CH_3$ | $Q_8$ | 6-Cl | 5-Cl | - | F=199-200° |

Wie vorstehend beschrieben, sind auch die folgenden Thioharnstoffe der Formel Ia′ erhältlich:

| $R_1$ | $R_2$ | $R_3$ | $R_5$ | $R_6$ | $R_7$ | $R_8$ |
|---|---|---|---|---|---|---|
| $C_5H_{11}(t)$ | $CH_3$ | $CH_3$ | $Q_1$ | Cl | H | – |
| $C_5H_{11}(t)$ | $CH_3$ | $CH_3$ | $Q_1$ | Cl | Cl | – |
| $C_5H_{11}(t)$ | $CH_3$ | $CH_3$ | $Q_1$ | Cl | Br | – |
| $C_5H_{11}(t)$ | $CH_3$ | $CH_3$ | $Q_1$ | Br | Br | – |
| $C_5H_{11}(t)$ | $C_2H_5$ | $C_2H_5$ | $Q_1$ | $CF_3$ | Br | – |
| $CH(i\ C_3H_7)_2$ | $CH_3$ | $CH_3$ | $Q_1$ | $CF_3$ | Br | – |
| $C_4H_9(t)$ | $C_2H_5$ | $CH_3$ | $Q_1$ | $CF_3$ | Br | – |
| 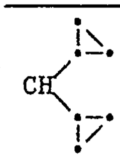 | $CH_3$ | $CH_3$ | $Q_1$ | $CF_3$ | Br | – |
| $C_4H_9(t)$ | $C_2H_5$ | $C_2H_5$ | $Q_4$ | $CH_3$ | – | – |
| $C_4H_9(t)$ | $C_3H_7(i)$ | $C_3H_7(i)$ | $Q_1$ | Cl | $CH_3$ | – |
| $C_5H_{11}(t)$ | $C_2H_5$ | $C_2H_5$ | $Q_1$ | Cl | Cl | – |
| $C_4H_9(s)$ | $C_2H_5$ | $C_2H_5$ | $Q_1$ | Cl | Cl | – |
| $C_3H_7(i)$ | $C_2H_5$ | $C_2H_5$ | $Q_1$ | Cl | Cl | – |
| 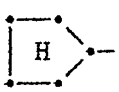 | $CH_3$ | $CH_3$ | $Q_1$ | Cl | $CH_3$ | – |
| 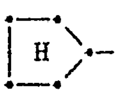 | $C_2H_5$ | $C_2H_5$ | $Q_1$ | Cl | $CH_3$ | – |
| $C_5H_{11}(t)$ | $CH_3$ | $CH_3$ | $Q_1$ | Cl | $CH_3$ | – |
| $C_5H_{11}(t)$ | $C_2H_5$ | $C_2H_5$ | $Q_1$ | Cl | $CH_3$ | – |
| $C_5H_{11}(t)$ | $C_3H_7(i)$ | $C_3H_7(i)$ | $Q_1$ | Cl | $CH_3$ | – |
| $C_3H_7(i)$ | $C_2H_5$ | $C_2H_5$ | $Q_1$ | Cl | $CH_3$ | – |

| $R_1$ | $R_2$ | $R_3$ | $R_5$ | $R_6$ | $R_7$ | $R_8$ |
|---|---|---|---|---|---|---|
| $C_4H_9(t)$ | $CH_3$ | $CH_3$ | $Q_1$ | Br | $CH_3$ | - |
| $C_4H_9(t)$ | $C_2H_5$ | $C_2H_5$ | $Q_1$ | Br | $CH_3$ | - |
| $C_4H_9(t)$ | $C_3H_7(i)$ | $C_3H_7(i)$ | $Q_1$ | Br | $CH_3$ | - |
| $C_4H_9(t)$ | $CH_3$ | $CH_3$ | $Q_1$ | $CH_3$ | $CH_3$ | - |
| $C_4H_9(t)$ | $C_2H_5$ | $C_2H_5$ | $Q_1$ | $CH_3$ | $CH_3$ | - |
| $C_4H_9(t)$ | $CH_3$ | $CH_3$ | $Q_4$ | $C_2H_5$ | - | - |
| $C_4H_9(t)$ | $C_2H_5$ | $C_2H_5$ | $Q_4$ | $C_2H_5$ | - | - |
| $C_4H_9(t)$ | $CH_3$ | $CH_3$ | $Q_4$ | $C_3H_7(i)$ | - | - |
| $C_4H_9(t)$ | $C_2H_5$ | $C_2H_5$ | $Q_4$ | $C_3H_7(i)$ | - | - |

In analoger Weise wie in Beispiel 3 angegeben werden auch die folgenden Thioharnstoffe der Formel Ia hergestellt:

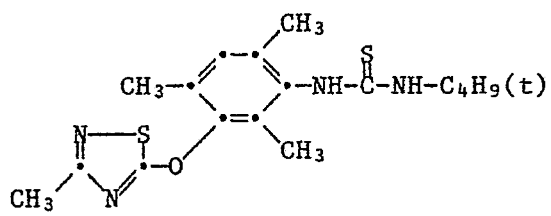

F = 145-147°C (Verbindung Nr. 3.50)

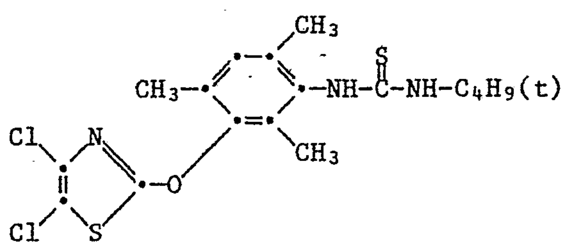

F = 157-158°C (Verbindung Nr. 3.51)

Beispiel 4: Herstellung von N-[2,6-Dimethyl-4-(4-trifluormethylthiazol-2-yloxy)-phenyl]-N'-tert.-butyl-S-methyl-isothioharnstoff

5 g N-[2,6-Dimethyl-4-(4-trifluormethyl-thiazol-2-yloxy)-phenyl]-N'-tert.butyl-thioharnstoff werden in 30 ml Aethanol und 2,1 g Jodmethan während 5 Stunden auf 50°C erwärmt. Der Alkohol wird anschliessend verdampft, der Rückstand in Methylenchlorid auf genommen und zweimal mit verdünnter Sodalösung gewaschen. Nach Trocknen über Natriumsulfat, Verdampfen des Lösungsmittels und Umkristallisieren in Hexan ergibt sich die Titelverbindung der Formel

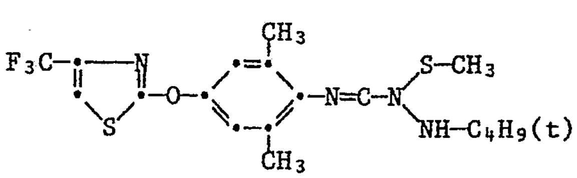

mit einem Smp. von 88-90°C (Verbindung Nr.4.01).

Entsprechend den vorstehend beschriebenen Arbeitsweisen werden die folgenden Verbindungen der Formel Ib'

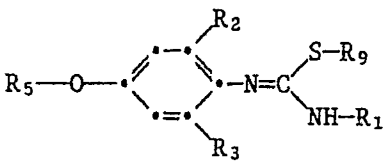

(Ib')

hergestellt:

| Verb.No | $R_1$ | $R_2$ | $R_3$ | $R_5$ | $R_6$ | $R_7$ | $R_8$ | $R_9$ | Phys. Dat. | Salzform |
|---|---|---|---|---|---|---|---|---|---|---|
| 4.01 | $C_4H_9$(t) | $CH_3$ | $CH_3$ | $Q_1$ | $CF_3$ | H | - | $CH_3$ | F= 88- 90° | - |
| 4.02 | $C_4H_9$(t) | $CH_3$ | $CH_3$ | $Q_1$ | $CF_3$ | Br | - | $CH_3$ | F=102-104° | - |
| 4.03 | $(CH_2)_3-OCH_3$ | $CH_3$ | $CH_3$ | $Q_1$ | $CF_3$ | Br | - | $CH_3$ | F= 53- 54° | - |
| 4.04 | $C_4H_9$(t) | $CH_3$ | $CH_3$ | $Q_1$ | $CF_3$ | Br | - | $C_2H_5$ | F= 89- 91° | - |
| 4.05 | 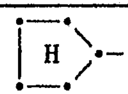 | $CH_3$ | $CH_3$ | $Q_1$ | $CF_3$ | Br | - | $CH_3$ | F=109-111° | - |
| 4.06 | 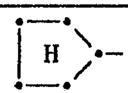 | $CH_3$ | $CH_3$ | $Q_1$ | $CF_3$ | H | - | $CH_3$ | F= 80- 81° | - |
| 4.07 | 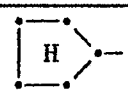 | $CH_3$ | $CH_3$ | $Q_1$ | $CF_3$ | H | - | $C_2H_5$ | F=75- 76° | - |
| 4.08 | 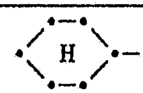 | $CH_3$ | $CH_3$ | $Q_1$ | $CF_3$ | H | - | $CH_3$ | F= 80- 81° | - |
| 4.09 | 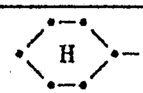 | $CH_3$ | $CH_3$ | $Q_1$ | $CF_3$ | H | - | $C_2H_5$ | F= 54- 56° | - |
| 4.10 | $C_5H_{11}$(t) | $CH_3$ | $CH_3$ | $Q_1$ | $CF_3$ | Br | - | $CH_3$ | F= 76- 78° | - |
| 4.11 | $C_5H_{11}$(t) | $CH_3$ | $CH_3$ | $Q_1$ | $CF_3$ | Br | - | $C_2H_5$ | F= 78- 80° | - |
| 4.12 | $C_4H_9$(t) | $CH_3$ | $CH_3$ | $Q_1$ | Cl | H | - | $CH_3$ | F=105-107° | - |
| 4.13 | $C_4H_9$(t) | $CH_3$ | $CH_3$ | $Q_1$ | Cl | H | - | $C_2H_5$ | F=101-103° | - |

| Verb.No | $R_1$ | $R_2$ | $R_3$ | $R_5$ | $R_6$ | $R_7$ | $R_8$ | $R_9$ | Phys. Dat. | Salzform |
|---|---|---|---|---|---|---|---|---|---|---|
| 4.14 | $C_4H_9$(t) | $CH_3$ | $CH_3$ | $Q_1$ | Br | H | - | $CH_3$ | F= 94- 96° | - |
| 4.15 | $C_4H_9$(t) | $CH_3$ | $CH_3$ | $Q_1$ | Br | H | - | $C_2H_5$ | F=105,5-107° | - |
| 4.16 | $C_4H_9$(t) | $CH_3$ | $CH_3$ | $Q_1$ | Cl | Cl | - | $CH_3$ | F=106,5-108° | - |
| 4.17 | $C_4H_9$(t) | $CH_3$ | $CH_3$ | $Q_1$ | Cl | Br | - | $CH_3$ | F=123-125°C | - |
| 4.18 | $C_4H_9$(t) | $CH_3$ | $CH_3$ | $Q_1$ | Br | Br | - | $CH_3$ | F=130,5-132° | - |
| 4.19 | $C_4H_9$(t) | $C_2H_5$ | $C_2H_5$ | $Q_1$ | $CF_3$ | Br | - | $CH_3$ | F= 92- 94° | - |
| 4.20 | $C_4H_9$(t) | $C_2H_5$ | $C_2H_5$ | $Q_1$ | Cl | Cl | - | $CH_3$ | F= 77,5-79° | - |
| 4.21 | $C_4H_9$(t) | $C_2H_5$ | $C_2H_5$ | $Q_1$ | Cl | Br | - | $CH_3$ | F= 77,5-79° | - |
| 4.22 | $C_4H_9$(t) | $CH_3$ | $CH_3$ | $Q_1$ | Br | Cl | - | $CH_3$ | F=117-119° | - |
| 4.23 | 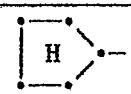 | $CH_3$ | $CH_3$ | $Q_1$ | $CF_3$ | Br | - | $C_2H_5$ | F= 90- 92° | |
| 4.24 | $C_4H_9$(t) | $C_2H_5$ | $C_2H_5$ | $Q_1$ | $CF_3$ | Br | - | $CH_3$ | F=167-170° | HJ |
| 4.25 | $C_4H_9$(t) | $CH_3$ | $CH_3$ | $Q_1$ | Br | Cl | - | $CH_3$ | F=193-195° | HJ |
| 4.26 | $C_4H_9$(t) | $CH_3$ | $CH_3$ | $Q_1$ | Br | H | - | $CH_3$ | F=179-180° | HJ |
| 4.27 | $C_4H_9$(t) | $CH_3$ | $CH_3$ | $Q_1$ | Cl | Br | - | $CH_3$ | F=191-192,5° | HJ |

| Verb.No | $R_1$ | $R_2$ | $R_3$ | $R_5$ | $R_6$ | $R_7$ | $R_8$ | $R_9$ | Phys. Dat. | Salzform |
|---|---|---|---|---|---|---|---|---|---|---|
| 4.28 | $C_4H_9(t)$ | $CH_3$ | $CH_3$ | $Q_1$ | Cl | Cl | – | $CH_3$ | F=175-177° | HJ |
| 4.29 | $C_4H_9(t)$ | $CH_3$ | $CH_3$ | $Q_1$ | Br | Br | – | $CH_3$ | F=199-201° | HJ |
| 4.30 | $C_4H_9(t)$ | $C_2H_5$ | $C_2H_5$ | $Q_1$ | Cl | Cl | – | $C_2H_5$ | $n_D^{24}$ =1,580 | – |
| 4.31 | $C_4H_9(t)$ | $C_2H_5$ | $C_2H_5$ | $Q_1$ | Cl | Br | – | $C_2H_5$ | $n_D^{24}$ =1,586 | – |
| 4.32 | $C_4H_9(t)$ | $C_2H_5$ | $C_2H_5$ | $Q_1$ | Cl | Br | – | $C_2H_5$ | F=177,5-180° | HJ |
| 4.33 | $C_4H_9(t)$ | $C_2H_5$ | $C_2H_5$ | $Q_1$ | Cl | Cl | – | $C_2H_5$ | F=170-172,5° | HJ |
| 4.34 | $C_4H_9(t)$ | $C_2H_5$ | $C_2H_5$ | $Q_1$ | Cl | Br | – | $CH_3$ | F=191,5-194° | HJ |
| 4.35 | $C_4H_9(t)$ | $C_2H_5$ | $C_2H_5$ | $Q_1$ | Cl | Cl | – | $CH_3$ | F=187-190° | HJ |
| 4.36 | $C_4H_9(t)$ | $CH_3$ | $CH_3$ | $Q_9$ | Cl | Cl | $CH_3$ | $CH_3$ | F=162-163,5° | – |
| 4.37 | $C_4H_9(t)$ | $CH_3$ | $CH_3$ | $Q_9$ | Cl | Cl | $CH_3$ | $CH_3$ | F=183,5-185° | HJ |
| 4.38 | $C_4H_9(t)$ | $C_2H_5$ | $C_2H_5$ | $Q_9$ | Cl | Cl | $CH_3$ | $CH_3$ | F= 81,5-83° | – |
| 4.39 | $C_4H_9(t)$ | $C_2H_5$ | $C_2H_5$ | $Q_9$ | Cl | Cl | $CH_3$ | $CH_3$ | F=154,5-156° | HJ |
| 4.40 | 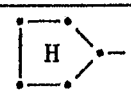 | $C_2H_5$ | $C_2H_5$ | $Q_1$ | Cl | Cl | – | $CH_3$ | F= 53- 54° | – |
| 4.41 | 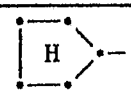 | $C_2H_5$ | $C_2H_5$ | $Q_1$ | Cl | Cl | – | $CH_3$ | F=190-192° | HJ |

| Verb.No | $R_1$ | $R_2$ | $R_3$ | $R_5$ | $R_6$ | $R_7$ | $R_8$ | $R_9$ | Phys. Dat. | Salzform |
|---|---|---|---|---|---|---|---|---|---|---|
| 4.42 | 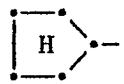 | $C_2H_5$ | $C_2H_5$ | $Q_1$ | Cl | Cl | - | $C_2H_5$ | $n_D^{23}$=1,5852 | - |
| 4.43 | 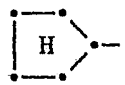 | $C_2H_5$ | $C_2H_5$ | $Q_1$ | Cl | Cl | - | $C_2H_5$ | F=187-188° | HJ |
| 4.44 | 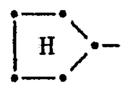 | $C_2H_5$ | $C_2H_5$ | $Q_1$ | Cl | Br | - | $CH_3$ | F= 66- 67° | - |
| 4.45 | 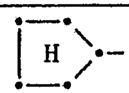 | $C_2H_5$ | $C_2H_5$ | $Q_1$ | Cl | Br | - | $CH_3$ | F= 190-192° | HJ |
| 4.46 | 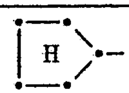 | $C_2H_5$ | $C_2H_5$ | $Q_1$ | Cl | Br | - | $C_2H_5$ | F= 47,5-49° | - |
| 4.47 | 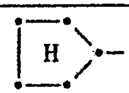 | $C_2H_5$ | $C_2H_5$ | $Q_1$ | Cl | Br | - | $C_2H_5$ | F=193-195° | HJ |
| 4.48 | $C_4H_9$(t) | $CH_3$ | $CH_3$ | $Q_2$ | $CF_3$ | - | $CH_3$ | $CH_3$ | F= 92- 93° | - |
| 4.49 | $C_4H_9$(t) | $CH_3$ | $CH_3$ | $Q_2$ | $CF_3$ | - | $C_3H_7$(i) | $CH_3$ | F=179-180° | - |
| 4.50 | $C_4H_9$(t) | $CH_3$ | $CH_3$ | $Q_2$ | $C_2F_5$ | - | $CH_3$ | $CH_3$ | F= 88- 89° | - |
| 4.51 | $C_4H_9$(t) | $CH_3$ | $CH_3$ | $Q_2$ | $C_2F_5$ | - | $C_3H_7$(i) | $CH_3$ | F=140-144° | - |
| 4.52 | $C_4H_9$(t) | $CH_3$ | $CH_3$ | $Q_2$ | $C_3F_7$(n) | - | $CH_3$ | $CH_3$ | F= 79- 80° | - |
| 4.53 | $C_4H_9$(t) | $C_2H_5$ | $C_2H_5$ | $Q_2$ | $C_3F_7$(n) | - | $CH_3$ | $CH_3$ | Oel | - |

| Verb.No | $R_1$ | $R_2$ | $R_3$ | $R_5$ | $R_6$ | $R_7$ | $R_8$ | $R_9$ | Phys. Dat. | Salzform |
|---|---|---|---|---|---|---|---|---|---|---|
| 4.54 | $C_4H_9$(t) | $C_2H_5$ | $C_2H_5$ | $Q_2$ | $CF_3$ | – | $CH_3$ | $CH_3$ | F= 95- 96° | – |
| 4.55 | $C_4H_9$(t) | $C_2H_5$ | $C_2H_5$ | $Q_2$ | $CF_3$ | – | $C_3H_7$(i) | $CH_3$ | F=127-128° | – |
| 4.56 | $C_4H_9$(t) | $C_2H_5$ | $C_2H_5$ | $Q_2$ | $C_2F_5$ | – | $CH_3$ | $CH_3$ | F= 77- 78° | – |
| 4.57 | $C_4H_9$(t) | $C_2H_5$ | $C_2H_5$ | $Q_2$ | $C_2F_5$ | – | $C_3H_7$(i) | $CH_3$ | F=112-113° | – |
| 4.58 | $C_4H_9$(t) | $CH_3$ | $CH_3$ | $Q_2$ | $C_3F_7$(n) | – | $C_3H_7$(i) | $CH_3$ | F= 96- 97° | – |
| 4.59 | $C_4H_9$(t) | $C_2H_5$ | $C_2H_5$ | $Q_2$ | $C_3F_7$(n) | – | $C_3H_7$(i) | $CH_3$ | F= 80- 81° | – |
| 4.60 | $C_4H_9$(t) | $CH_3$ | $CH_3$ | $Q_3$ | $CF_3$ | – | – | $CH_3$ | F= 83- 85° | – |
| 4.61 | $C_3H_7$(i) | $CH_3$ | $CH_3$ | $Q_3$ | $CF_3$ | – | – | $CH_3$ | F= 92- 94° | – |
| 4.62 | $C_4H_9$(t) | $C_3H_7$(i) | $C_3H_7$(i) | $Q_4$ | $CH_3$ | – | – | $CH_3$ | F= 93- 95° | – |
| 4.63 | $C_4H_9$(t) | $CH_3$ | $CH_3$ | $Q_1$ | Cl | $CH_3$ | – | $CH_3$ | F=127,5-129° | – |
| 4.64 | $C_4H_9$(t) | $CH_3$ | $CH_3$ | $Q_1$ | Cl | $CH_3$ | – | $CH_3$ | F=162-166° | HJ |
| 4.65 | $C_4H_9$(t) | $C_2H_5$ | $C_2H_5$ | $Q_1$ | Cl | $CH_3$ | – | $CH_3$ | F= 83- 84° | – |
| 4.66 | $C_4H_9$(t) | $C_2H_5$ | $C_2H_5$ | $Q_1$ | Cl | $CH_3$ | – | $CH_3$ | F=165-168° | HJ |
| 4.67 | $C_4H_9$(t) | $C_3H_7$(i) | $C_3H_7$(i) | $Q_1$ | Cl | Cl | – | $CH_3$ | F= 93- 97° | HJ |
| 4.68 | $C_4H_9$(t) | $C_3H_7$(i) | $C_3H_7$(i) | $Q_1$ | Cl | Cl | – | $CH_3$ | $n_D^{27}$ = 1,5608 | – |

| Verb.No | $R_1$ | $R_2$ | $R_3$ | $R_5$ | $R_6$ | $R_7$ | $R_8$ | $R_9$ | Phys. Dat. | Salzform |
|---|---|---|---|---|---|---|---|---|---|---|
| 4.69 | 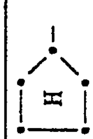 | $C_3H_7(i)$ | $C_3H_7(i)$ | $Q_1$ | Cl | Cl | - | $CH_3$ | F=170-174° | HJ |
| 4.70 | 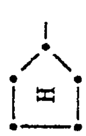 | $C_3H_7(i)$ | $C_3H_7(i)$ | $Q_1$ | Cl | Cl | - | $CH_3$ | F= 93-96,5° | - |
| 4.71 | $C_4H_9(t)$ | $CH_3$ | $CH_3$ | $Q_8$ | 6-Cl | 5-Cl | - | $CH_3$ | F=160-164°*) | HJ |
| 4.72 | $C_4H_9(t)$ | $CH_3$ | $CH_3$ | $Q_8$ | 6-Cl | 5-Cl | - | $CH_3$ | F= 98-104°*) | - |
| 4.73 | $C_3H_7(i)$ | $CH_3$ | $CH_3$ | $Q_8$ | 6-Cl | 5-Cl | - | $CH_3$ | F=187-191°*) | HJ |
| 4.74 | $C_3H_7(i)$ | $CH_3$ | $CH_3$ | $Q_8$ | 6-Cl | 5-Cl | - | $CH_3$ | F=160-163,5°*) | - |

*) enthält geringe Anteile weiterer Isomeren

Wie vorstehend beschrieben, sind auch folgende Isothioharnstoffe der Formel Ib' darstellbar:

| $R_1$ | $R_2$ | $R_3$ | $R_5$ | $R_6$ | $R_7$ | $R_8$ | $R_9$ |
|---|---|---|---|---|---|---|---|
| $C_4H_9(t)$ | $CH_3$ | $CH_3$ | $Q_1$ | Cl | Cl | – | $C_2H_5$ |
| $C_4H_9(t)$ | $CH_3$ | $CH_3$ | $Q_1$ | Cl | Br | – | $C_2H_5$ |
| $C_4H_9(t)$ | $CH_3$ | $CH_3$ | $Q_1$ | Br | Br | – | $C_2H_5$ |
| $C_5H_{11}(t)$ | $CH_3$ | $CH_3$ | $Q_1$ | Cl | H | – | $CH_3$ |
| $C_5H_{11}(t)$ | $CH_3$ | $CH_3$ | $Q_1$ | Cl | Cl | – | $CH_3$ |
| $C_5H_{11}(t)$ | $CH_3$ | $CH_3$ | $Q_1$ | Cl | Br | – | $CH_3$ |
| $C_5H_{11}(t)$ | $CH_3$ | $CH_3$ | $Q_1$ | Br | H | – | $CH_3$ |
| $C_5H_{11}(t)$ | $CH_3$ | $CH_3$ | $Q_1$ | Br | Br | – | $CH_3$ |
| $C_4H_9(t)$ | $C_2H_5$ | $C_2H_5$ | $Q_1$ | $CF_3$ | Br | – | $C_2H_5$ |
| $C_5H_{11}(t)$ | $C_2H_5$ | $C_2H_5$ | $Q_1$ | $CF_3$ | Br | – | $CH_3$ |
| $CH(i\text{-}C_3H_7)_2$ | $CH_3$ | $CH_3$ | $Q_1$ | $CF_3$ | Br | – | $CH_3$ |
| 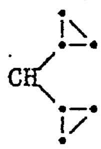 | $CH_3$ | $CH_3$ | $Q_1$ | $CF_3$ | Br | – | $CH_3$ |
| $C_4H_9(t)$ | $C_2H_5$ | $C_2H_5$ | $Q_4$ | $CH_3$ | – | – | $CH_3$ |
| $C_4H_9(t)$ | $C_2H_5$ | $C_2H_5$ | $Q_4$ | $CH_3$ | – | – | $C_2H_5$ |
| $C_4H_9(t)$ | $C_3H_7(i)$ | $C_3H_7(i)$ | $Q_1$ | Cl | $CH_3$ | – | $CH_3$ |
| $C_5H_{11}(t)$ | $C_2H_5$ | $C_2H_5$ | $Q_1$ | Cl | Cl | – | $CH_3$ |
| $C_4H_9(s)$ | $C_2H_5$ | $C_2H_5$ | $Q_1$ | Cl | Cl | – | $CH_3$ |
| $C_3H_7(i)$ | $C_2H_5$ | $C_2H_5$ | $Q_1$ | Cl | Cl | – | $CH_3$ |
| 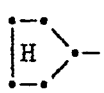 | $CH_3$ | $CH_3$ | $Q_1$ | Cl | $CH_3$ | – | $CH_3$ |

| $R_1$ | $R_2$ | $R_3$ | $R_5$ | $R_6$ | $R_7$ | $R_8$ | $R_9$ |
|---|---|---|---|---|---|---|---|
| 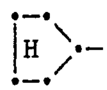 | $C_2H_5$ | $C_2H_5$ | $Q_1$ | Cl | $CH_3$ | - | $CH_3$ |
| $C_5H_{11}(t)$ | $CH_3$ | $CH_3$ | $Q_1$ | Cl | $CH_3$ | - | $CH_3$ |
| $C_5H_{11}(t)$ | $C_2H_5$ | $C_2H_5$ | $Q_1$ | Cl | $CH_3$ | - | $CH_3$ |
| $C_9H_{11}(t)$ | $C_3H_7(i)$ | $C_3H_7(i)$ | $Q_1$ | Cl | $CH_3$ | - | $CH_3$ |
| $C_3H_7(t)$ | $C_2H_5$ | $C_2H_5$ | $Q_1$ | Cl | $CH_3$ | - | $CH_3$ |
| $C_4H_9(t)$ | $CH_3$ | $CH_3$ | $Q_1$ | Br | $CH_3$ | - | $CH_3$ |
| $C_4H_9(t)$ | $C_2H_5$ | $C_2H_5$ | $Q_1$ | Br | $CH_3$ | - | $CH_3$ |
| $C_4H_9(t)$ | $C_3H_7(i)$ | $C_3H_7(i)$ | $Q_1$ | Br | $CH_3$ | - | $CH_3$ |
| $C_4H_9(t)$ | $CH_3$ | $CH_3$ | $Q_1$ | $CH_3$ | $CH_3$ | - | $CH_3$ |
| $C_4H_9(t)$ | $C_2H_5$ | $C_2H_5$ | $Q_1$ | $CH_3$ | $CH_3$ | - | $CH_3$ |
| $C_4H_9(t)$ | $CH_3$ | $CH_3$ | $Q_4$ | $C_2H_5$ | - | - | $CH_3$ |
| $C_4H_9(t)$ | $C_2H_5$ | $C_2H_5$ | $Q_4$ | $C_2H_5$ | - | - | $CH_3$ |
| $C_4H_9(t)$ | $CH_3$ | $CH_3$ | $Q_4$ | $C_3H_7(i)$ | - | - | $CH_3$ |
| $C_4H_9(t)$ | $C_2H_5$ | $C_2H_5$ | $Q_4$ | $C_3H_7(i)$ | - | - | $CH_3$ |

Gemäss dem Verfahren von Beispiel 4 wird auch der folgende, unter Formel Ib fallende Isothioharnstoff erhalten:

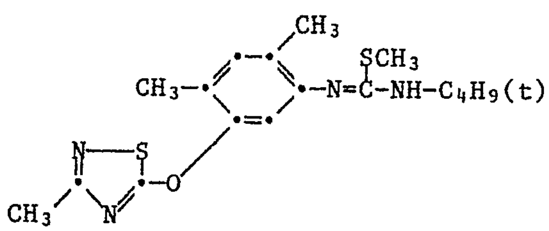

F = 91- 93°C
(Verbindung Nr. 4.75)

Beispiel 5: Herstellung von N-[2,6-Diäthyl-4-(4,5-dichlor-thiazol-2-yl)-oxy]-phenyl-N'-(t-butyl)-carbodiimid

5,0 g N-[2,6-Diäthyl-4-(4,5-dichlor-thiazol-2-yl)-oxy]-phenyl-N'-(tert.butyl)-thioharnstoff und 3,55 g 2-Chlor-1-methypyridiniumjodid werden in 35 ml trockenem Acetonitril vorgelegt und bei Rückflusstemperatur werden 2,8 g Triäthylamin in 50 ml Acetonitril zugetropft. Anschliessend wird noch etwa 25 Stunden am Rückfluss gerührt, das Lösungsmittel i.Vak. entfernt und der Rückstand in Hexan aufgenommen. Nach dem Abfiltrieren vom Unlöslichen wird das Filtrat dreimal mit Wasser gewaschen, über $Na_2SO_4$ getrocknet und das Lösungsmittel i.Vak. entfernt.

Man isoliert die Titelverbindung der Formel

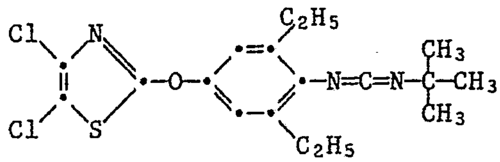

als gelbes Oel mit einem Brechungsindex von $n_D^{24}$ = 1,5783, (Verbindung Nr. 5.09).

Entsprechend den vorstehend beschriebenen Arbeitsweisen werden die folgenden Carbodiimide der Formel Ic'

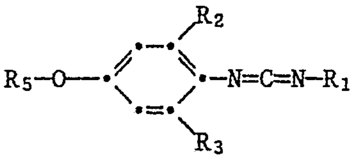

(Ic')

hergestellt:

| Verb.No | $R_1$ | $R_2$ | $R_3$ | $R_5$ | $R_6$ | $R_7$ | $R_8$ | Phys. Dat. |
|---|---|---|---|---|---|---|---|---|
| 5.01 | $C_4H_9$(t) | $CH_3$ | $CH_3$ | $Q_1$ | $CF_3$ | H | – | F= 101-102° |
| 5.02 | $C_4H_9$(t) | $CH_3$ | $CH_3$ | $Q_1$ | $CF_3$ | Br | – | F= 53- 55° |
| 5.03 | $C_5H_{11}$(t) | $CH_3$ | $CH_3$ | $Q_1$ | $CF_3$ | Br | – | Oel*) |
| 5.04 | $C_4H_9$(t) | $CH_3$ | $CH_3$ | $Q_1$ | Cl | Cl | – | $n_D^{24}$=1,5868 |
| 5.05 | $C_4H_9$(t) | $CH_3$ | $CH_3$ | $Q_1$ | Cl | Br | – | $n_D^{23}$=1,5978 |
| 5.06 | $C_4H_9$(t) | $CH_3$ | $CH_3$ | $Q_1$ | Br | Br | – | $n_D^{24}$=1,6106 |
| 5.07 | $C_4H_9$(t) | $C_2H_5$ | $C_2H_5$ | $Q_1$ | $CF_3$ | Br | – | $n_D^{24}$=1,5476 |
| 5.08 | $C_4H_9$(t) | $CH_3$ | $CH_3$ | $Q_1$ | Br | Cl | – | $n_D^{24}$=1,5983 |
| 5.09 | $C_4H_9$(t) | $C_2H_5$ | $C_2H_5$ | $Q_1$ | Cl | Cl | – | $n_D^{24}$=1,5783 |
| 5.10 | $C_4H_9$(t) | $C_2H_5$ | $C_2H_5$ | $Q_1$ | Cl | Br | – | $n_D^{24}$=1,5892 |
| 5.11 | $C_4H_9$(t) | $CH_3$ | $CH_3$ | $Q_2$ | $CF_3$ | – | $CH_3$ | F= 61- 64° |
| 5.12 | $C_4H_9$(t) | $CH_3$ | $CH_3$ | $Q_2$ | $CF_3$ | – | $C_3H_7$(i) | F= 81- 82° |
| 5.13 | $C_4H_9$(t) | $CH_3$ | $CH_3$ | $Q_2$ | $C_2F_5$ | – | $CH_3$ | F= 55- 57° |
| 5.14 | $C_4H_9$(t) | $CH_3$ | $CH_3$ | $Q_2$ | $C_3F_7$(n) | – | $CH_3$ | Oel*) |
| 5.15 | $C_4H_9$(t) | $CH_3$ | $CH_3$ | $Q_3$ | $C_4H_9$(t) | – | – | F= 74- 76° |

*) Struktur entspricht dem Protonenresonanz-Spektrum

| Verb.No | $R_1$ | $R_2$ | $R_3$ | $R_5$ | $R_6$ | $R_7$ | $R_8$ | Phys. Dat. |
|---|---|---|---|---|---|---|---|---|
| 5.16 | $C_4H_9$(t) | $C_2H_5$ | $C_2H_5$ | $Q_9$ | Cl | Cl | $CH_3$ | $n_D^{23}$ = 1,5615 |
| 5.17 | $C_4H_9$(t) | $CH_3$ | $CH_3$ | $Q_9$ | Cl | Cl | $CH_3$ | F= 79,5-71,5° |
| 5.18 | 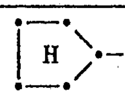 | $C_2H_5$ | $C_2H_5$ | $Q_1$ | Cl | Cl | - | $n_D^{23}$ = 1,5938 |
| 5.19 | 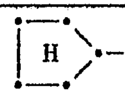 | $C_2H_5$ | $C_2H_5$ | $Q_1$ | Cl | Br | - | $D_D^{22}$ = 1,6013 |
| 5.20 | $C_4H_9$(t) | $CH_3$ | $CH_3$ | $Q_5$ | $C_4H_9$(t) | - | - | Oel*) |
| 5.21 | $C_4H_9$(t) | $CH_3$ | $CH_3$ | $Q_2$ | $C_2F_5$ | - | $C_3H_7$(i) | F= 70- 71° |
| 5.22 | $C_4H_9$(t) | $CH_3$ | $CH_3$ | $Q_2$ | $C_3F_7$(n) | - | $C_3H_7$(i) | F= 64- 65° |
| 5.23 | $C_4H_9$(t) | $C_2H_5$ | $C_2H_5$ | $Q_2$ | $CF_3$ | - | $C_3H_7$(i) | F= 50- 51° |
| 5.24 | $C_4H_9$(t) | $C_2H_5$ | $C_2H_5$ | $Q_2$ | $C_2F_5$ | - | $CH_3$ | Oel*) |
| 5.25 | $C_4H_9$(t) | $C_2H_5$ | $C_2H_5$ | $Q_2$ | $C_2F_5$ | - | $C_3H_7$(i) | F= 46- 47° |
| 5.26 | $C_4H_9$(t) | $C_2H_5$ | $C_2H_5$ | $Q_2$ | $C_3F_7$(n) | - | $CH_3$ | Oel*) |
| 5.27 | $C_4H_9$(t) | $C_2H_5$ | $C_2H_5$ | $Q_2$ | $C_3F_7$(n) | - | $C_3H_7$(i) | F= 39- 41° |
| 5.28 | $C_4H_9$(t) | $C_2H_5$ | $C_2H_5$ | $Q_2$ | $CF_3$ | - | $CH_3$ | F= 43- 44° |
| 5.29 | $C_4H_9$(t) | $C_2H_5$ | $C_2H_5$ | $Q_3$ | $C_4H_9$(t) | - | - | Oel*) |

*) Struktur entspricht dem Protonenresonanz-Spektrum

| Verb.No | $R_1$ | $R_2$ | $R_3$ | $R_5$ | $R_6$ | $R_7$ | $R_8$ | Phys. Dat. |
|---|---|---|---|---|---|---|---|---|
| 5.30 | $C_4H_9(t)$ | $CH_3$ | $CH_3$ | $Q_3$ | $CF_3$ | - | - | $n_D^{20}$ = 1,5340 |
| 5.31 | $C_3H_7(i)$ | $CH_3$ | $CH_3$ | $Q_3$ | $CF_3$ | - | - | $n_D^{20}$ = 1,5406 |
| 5.32 | $C_4H_9(t)$ | $CH_3$ | $CH_3$ | $Q_4$ | $CH_3$ | - | - | $n_D^{25}$ = 1,5685 |
| 5.33 | $C_3H_7(i)$ | $CH_3$ | $CH_3$ | $Q_4$ | $CH_3$ | - | - | $n_D^{40}$ = 1,5716 |
| 5.34 | $C_4H_9(t)$ | $CH_3$ | $CH_3$ | $Q_9$ | Cl | Cl | $CH_3$ | F= 70,5-71,5° |
| 5.35 | $C_4H_9(t)$ | $C_2H_5$ | $C_2H_5$ | $Q_9$ | Cl | Cl | $CH_3$ | $n_D^{23}$ = 1,5615 |
| 5.36 | $C_4H_9(t)$ | $CH_3$ | $CH_3$ | $Q_1$ | Cl | $CH_3$ | - | $n_D^{26}$ = 1,5788 |
| 5.37 | $C_4H_9(t)$ | $C_2H_5$ | $C_2H_5$ | $Q_1$ | Cl | $CH_3$ | - | $n_D^{26}$ = 1,5693 |
| 5.38 | $C_4H_9(t)$ | $C_3H_7(i)$ | $C_3H_7(i)$ | $Q_1$ | Cl | Cl | - | $n_D^{27}$ = 1,5660 |
| 5.39 | 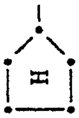 | $C_3H_7(i)$ | $C_3H_7(i)$ | $Q_1$ | Cl | Cl | - | $n_D^{27}$ = 1,5798 |
| 5.40 | $C_3H_7(i)$ | $CH_3$ | $CH_3$ | $Q_8$ | 6-Cl | 5-Cl | - | F= 66-71° |
| 5.41 | $C_4H_9(t)$ | $CH_3$ | $CH_3$ | $Q_8$ | 6-Cl | 5-Cl | - | F= 56-67° |

*) Struktur entspricht dem Protonenresonanz-Spektrum

In analoger Weise wie vorstehend im Beispiel 5 beschrieben sind auch die folgenden Carbodiimide der Formel Ic' herstellbar:

| $R_1$ | $R_2$ | $R_3$ | $R_5$ | $R_6$ | $R_7$ | $R_8$ |
|---|---|---|---|---|---|---|
| $C_4H_9(t)$ | $C_3H_7(i)$ | $C_3H_7(i)$ | $Q_1$ | Cl | $CH_3$ | - |
| $C_5H_{11}(t)$ | $C_2H_5$ | $C_2H_5$ | $Q_1$ | Cl | Cl | - |
| $C_4H_9(s)$ | $C_2H_5$ | $C_2H_5$ | $Q_1$ | Cl | Cl | - |
| $C_3H_7(i)$ | $C_2H_5$ | $C_2H_5$ | $Q_1$ | Cl | Cl | - |
| 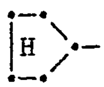 | $CH_3$ | $CH_3$ | $Q_1$ | Cl | $CH_3$ | - |
| 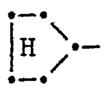 | $C_2H_5$ | $C_2H_5$ | $Q_1$ | Cl | $CH_3$ | - |
| $C_5H_{11}(t)$ | $CH_3$ | $CH_3$ | $Q_1$ | Cl | $CH_3$ | - |
| $C_5H_{11}(t)$ | $C_2H_5$ | $C_2H_5$ | $Q_1$ | Cl | $CH_3$ | - |
| $C_5H_{11}(t)$ | $C_3H_7(i)$ | $C_3H_7(i)$ | $Q_1$ | Cl | $CH_3$ | - |
| $C_3H_7(i)$ | $C_2H_5$ | $C_2H_5$ | $Q_1$ | Cl | $CH_3$ | - |
| $C_4H_9(t)$ | $CH_3$ | $CH_3$ | $Q_1$ | Br | $CH_3$ | - |
| $C_4H_9(t)$ | $C_2H_5$ | $C_2H_5$ | $Q_1$ | Br | $CH_3$ | - |
| $C_4H_9(t)$ | $C_3H_7(i)$ | $C_3H_7(i)$ | $Q_1$ | Br | $CH_3$ | - |
| $C_4H_9(t)$ | $CH_3$ | $CH_3$ | $Q_1$ | $CH_3$ | $CH_3$ | - |
| $C_4H_9(t)$ | $C_2H_5$ | $C_2H_5$ | $Q_1$ | $CH_3$ | $CH_3$ | - |
| $C_4H_9(t)$ | $CH_3$ | $CH_3$ | $Q_4$ | $C_2H_5$ | - | - |
| $C_4H_9(t)$ | $C_2H_5$ | $C_2H_5$ | $Q_4$ | $C_2H_5$ | - | - |
| $C_4H_9(t)$ | $CH_3$ | $CH_3$ | $Q_4$ | $C_3H_7(i)$ | - | - |
| $C_4H_9(t)$ | $C_2H_5$ | $C_2H_5$ | $Q_4$ | $C_3H_7(i)$ | - | - |
| 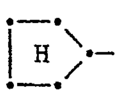 | $CH_3$ | $CH_3$ | $Q_1$ | $CF_3$ | H | - |

| $R_1$ | $R_2$ | $R_3$ | $R_5$ | $R_6$ | $R_7$ | $R_8$ |
|---|---|---|---|---|---|---|
| 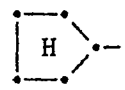 | $CH_3$ | $CH_3$ | $Q_1$ | $CF_3$ | Br | - |
| 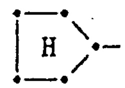 | $CH_3$ | $CH_3$ | $Q_1$ | $CF_3$ | H | - |
| $C_4H_9(t)$ | $CH_3$ | $CH_3$ | $Q_1$ | Br | H | - |
| $C_5H_{11}(t)$ | $CH_3$ | $CH_3$ | $Q_1$ | Br | H | - |

In analoger Weise wie in Beispiel 5 angegeben werden auch die folgenden Carbodiimide der Formel Ic hergestellt:

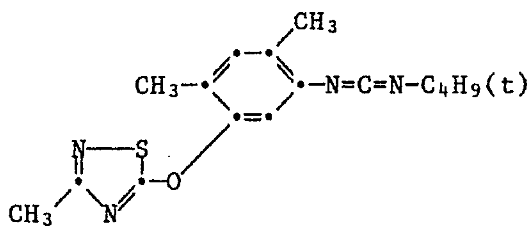

$n_D^{25} = 1,5632$
(Verbindung Nr. 5.42)

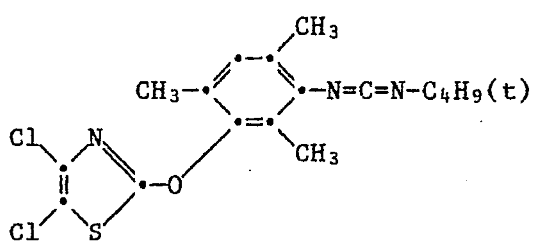

helles Oel
(Verbindung Nr. 5.43)

Beispiel 6: Wirkung gegen Tetranychus urticae (OP-sensible) und Tetranychus cinnabarinus (OP-tolerant.)

Die Primärblätter von Phaseolus vulgaris-Pflanzen wurden 12 Stunden vor dem Versuch auf akarizide Wirkung mit einem infestierten Blattstück aus einer Massenzucht von Tetranychus urticae (OP-sens.) oder Tetranychus cinnabarinus (OP-tol.) belegt (Mischpopulation). Die Toleranz bezieht sich auf die Verträglichkeit gegenüber Diazinon.

Die so behandelten infestierten Pflanzen werden mit einer Versuchslösung im Emulsionsform enthaltend 400 ppm der zu prüfenden Verbindung bis zur Tropfnässe besprüht.

Nach 24 Stunden und wiederum nach 6 (T.urticae) bzw. 7 Tagen (T.cinnabarinus) werden Imagines und Larven (alle beweglichen Stadien) sowie abgelegte Eier unter dem Binokular auf lebende und tote Individuen ausgewertet.

Man verwendet pro Testspezies eine Pflanze. Während des Versuchsverlaufs stehen die Pflanzen in Gewächshauskabinen bei 25°C mit etwa 50-60 % rel. Luftfeuchtigkeit.

Verbindungen der Formel I gemäss Beispiel 3, 4 und 5 zeigen in diesem Versuch gute Wirkung gegen Tetranychus urticae und Tetranychus cinnabarinus.

Beispiel 7: Ovizide Wirkung auf Tetranychus urticae (OP-resistent)

Eingetopfte Phaseolus vulgaris-Pflanzen im Primärblatt-Stadium werden jeweils zweimal mit 30 Weibchen von Tetranychus urticae besiedelt. Nach 24-stündiger Eiablage werden die Weibchen mit einer Saugpumpe (Wasserstrahlpumpe) von den Pflanzen entfernt, so dass auf diesen nur noch die abgelegten Milbeneier verbleiben.

Die mit den Milbeneiern infestierten Pflanzen werden dann mit einer wässrigen Emulsion enthaltend 400

ppm der zu prüfenden Verbindung bis zur Tropfnässe besprüht und 5 Tage bie 25°C und etwa 50 % relativer Luftfeuchtigkeit gehalten. Nach dieser Zeit wird die prozentuale Mortalität der Eier und geschlüpften Larven durch Auszählen bestimmt.

Verbindungen der Formel I gemäss dem vorstehenden Beispielen 3, 4 und 5 zeigen gute Wirkung in obigem Test.

Beispiel 8: Pflanzenmitizide Blattpenetrations-Wirkung auf Tetranychus cinnabarinus

Für den Versuch werden eingetopfte Buschbohnenpflanzen im Primärblatt-Stadium, die mit Tetranychus cinnabarinus infestiert sind, verwendet. Die Besiedlung mit den Milben erfolgt 1 Tag vor der Wirkstoffapplikation.

Die Blattoberseiten der mit diesen Milben infestierten Versuchspflanzen werden mit einer Emulsionszubereitung enthaltend 400 ppm des zu prüfenden Wirkstoffes besprüht. Nach dem Antrocknen des Sprühbelages wird jeweils der Rand der Blattoberseite einer Anzahl befallener Blätter mit einem Wulst aus zähflüssigem Leim (Raupenleim) abgegrenzt, um ein Ueberlaufen der Milben von der Blattunterseite auf die Blattoberseite zu verhindern.

Die behandelten Pflanzen werden dann im Gewächshaus bei einer Temperatur von 25°C bis 27°C und einer relativen Luftfeuchtigkeit von ca. 50% gehalten. Sechs Tage nach der Wirkstoffapplikation wird festgestellt, ob eine translaminare Wirkung, d.h. Blattpenetration des Wirkstoffs von der Blattoberseite zur Blattunterseite, eingetreten war, und zwar durch Bestimmung der prozentualen Mortalität der Eier und larvalen sowie adulten Stadien.

Verbindungen der Formel I gemäss den vorstehenden Beispielen 3, 4 und 5 zeigen gute Wirkung in obigem Test.

Beispiel 9: Wirkung gegen Panonychus ulmi (OP und Carb. resistent)

Eingetopfte Apfelsämlinge mit etwa 20-30 Blättern werden mit jeweils 60 adulten Weibchen von Panonychus ulmi besiedelt. Nach sieben Tagen werden die infestierten Pflanzen mit einer wässrigen Emulsion enthaltend 400 ppm der zu prüfenden Verbindung bis zur Tropfnässe besprüht. Die behandelten Pflanzen werden dann während weiterer 14 Tage bei 25°C und etwa 50 % relativer Luftfeuchtigkeit im Gewächshaus aufgestellt.

Nach dieser Zeit erfolgt die Auswertung, indem man 20 Blätter pro Pflanze entnimmt, die Milbenpopulation mittels einer Abbürst-Vorrichtung von den entnommenen Blättern entfernt und unter dem Binocular nach Eiern, postembryonalen Stadien und Adulten auszählt. Bewertet wird die prozentuale Reduktion der Milbenpopulation gegenüber unbehandelten Kontrollen.

Verbindungen der Formel I gemäss den vorstehenden Beispielen 3, 4 und 5 zeigen gute Wirkung in obigem Test.

Beispiel 10: Wirkung gegen tierparasitäre Milben

Von mit Dermanyssus gallinae befallenen Hühnern werden Ansätze bestend aus etwa 50 Milben verschiedener Stadien (Mischpopulation: Larven, Nymphen und Adulte) entnommen. Die Milbenansätze werden jeweils mit einer wässrigen Emulsion, Suspension bzw. Lösung enthaltend 800 ppm des zur prüfenden Wirkstoffes benetzt. Hierzu werden die Milbenansätze in einem Reagenzglas mit der den Wirkstoff enthaltenden flüssigen Zubereitung übergossen; die Flüssigkeit wird anschliessend mit Hilfe eines Wattebausches aufgesogen. Die so benetzten und behandelten Milben verbleiben während 72 Stunden in dem Reagenzglas. Nach dieser Zeit wird die Abtötung der behandelten Milben gegenüber unbehandelten Kontrollansätzen ermittelt.

Verbindungen der Formel I gemäss den vorstehenden Beispielen 3, 4 und 5 zeigen gute Wirkung im obigen Test.

Beispiel 11: Wirkung gegen Zecken: Abtötungswirkung in verschiedenen Entwicklungsstadien

Als Testobjekte pro Ansatz werden nicht vollgesogene Larven (jeweils ca. 50), Nymphen (jeweils 5) oder Adulte (jeweils 5) der Zeckenarten Rhipicephalus bursa, Amblyomma hebraeum und Boophilus microplus verwendet. Die Testtiere (in der angegebenen Anzahl) werden für kurze Zeit in 2 bis 3 ml einer wässriger Emulsion der zu untersuchenden Substanzen mit einer Konzentration von 400 ppm getaucht, die sich in einem Reagenzglas befindet. Die Reagenzgläser werden dann mit einem Wattepfropfen verschlossen und 10 Minuten nach dem Eintauchen der Testtiere geschüttelt. Dabei wird die Wirkstoff-Emulsion von den Wattepfropfen aufgesogen und die benetzten Testtiere in den so kontaminierten Reganzgläsern belassen. Die Auswertung (% - Mortalität) erfolgt für Larven nach 3 Tagen und für Nymphen und Adulte nach 14 Tagen.

Verbindungen der Formel I gemäss den vorstehenden Beispielen 3, 4 und 5 zeigen gute Wirkung im obigen Test.

Beispiel 12: Wirkung gegen Zecken: Eiablagehemmung

Als Testtiere werden vollgesogene adulte Weibchen der Rinderzecke Boophilus microplus verwendet. Es werden je 10 Zecken eines OP-resistenten Stammes (z.B. vom Biarra-Stamm) und eines normal empfindlichen Stammes (z.B. vom Yeerongpilly-Stamm) behandelt. Die Zecken werden auf mit Doppelklebeband bezogenen Platten fixiert und dann entweder mit wässrigen Emulsionen bzw. mit Lösungen enthaltend 800 ppm der zu untersuchenden Verbindung oder eines ihrer Salze benetzt oder mit einem mit diesen Flüssigkeiten

getränkten Wattebausch in Berühung gebracht. Anschliessend werden sie in einem klimatisierten Raum bei konstanten Bedingungen aufbewahrt. Die Auswertung erfolgt nach drei Wochen. Es wird die prozentuale Hemmung der Ablage von fertilen Eiern gegenüber unbehandelten Kontrollen ermittelt.

Verbindungen der Formel I gemäss den vorstehenden Beispielen 3, 4 und 5 zeigen gute Wirkung im obigen Test.

Beispiel 13: Wirkung gegen Musca domestica

Je 50 g frisch zubereitetes Nährsubstrat für Maden werden in Becher eingewogen. Von einer 1 Gew.-%igen acetonischen Lösung des betreffenden Wirkstoffes wird eine bestimmte Menge auf das in den Bechern befindliche Nährsubstrat pipettiert, so dass sich eine Wirkstoffkonzentration von 400 ppm ergibt. Nach dem Durchmischen des Substrates lässt man das Aceton mindestens 20 Stunden lang verdampfen.

Dann werden pro Wirkstoff und Konzentration je 25 eintägige Maden von Musca domestica in die das so behandelte Nährsubstrat enthaltenden Becher gegeben. Nachdem sich die Maden verpuppt haben, werden die gebildeten Puppen durch Ausschwemmen mit Wasser von dem Substrat abgetrennt und in mit Siebdeckeln verschlossenen Gefässen deponiert.

Die pro Ansazt ausgeschwemmten Puppen werden gezählt (toxischer Einfluss des Wirkstoffes auf die Madenentwicklung). Dann wird nach 10 Tagen die Anzahl der aus den Puppen geschlüpften Fliegen bestimmt.

Verbindungen der Formel I gemäss den vorstehenden Beispielen 3, 4 und 5 zeigen gute Wirkung im obigen Test.

Beispiel 14: Insektizide Frassgift-Wirkung

Baumwollpflanzen (ca. 20 cm hoch) werden mit wässrigen Wirkstoffemulsionen (erhalten aus einen 10%igen emulgierbaren Konzentrat) besprüht, wobei die Wirkstoffemulsionen 400 ppm der jeweils zu prüfenden Verbindung enthalten.

Nach dem Antrocknen des Belages werden die Baumwollpflanzen mit Spodoptera littoralis- und Heliothis virescens-Larven im dritten larvalen Stadium besetzt. Der Versuch wird bei 24°C und 60 % relativer Luftfeuchtigkeit durchgeführt. In Abständen von jeweils 48 und 96 Stunden werden Mortalität sowie Entwicklungs- und Häutungsstörungen der angesetzten Larven bestimmt.

Verbindungen der Formel I gemäss den vorstehenden Beispielen 3, 4 und 5 zeigen gute Wirkung im obigen Test.

Beispiel 15: Wirkung gegen Lucilia sericata

Zu 9 ml eines Zuchtmediums wird bei 50°C 1 ml einer 0,5 % Aktivsubstanz enthaltenden wässrigen Zubereitung gegeben. Nun werden ca. 30 frisch geschlüpfte Lucilia sericata-Larven zum Zuchtmedium hinzugefügt. Nach 48 und 96 Stunden wird die insektizide Wirkung durch Emittlung der Abtötungsrate festgestellt.

Verbindungen der Formel I gemäss den vorstehenden Beispielen 3, 4 und 5 zeigen gute Wirkung im obigen Test.

Beispiel 16: Wirkung gegen Aëdes aegypti

Auf die Oberfläche von 150 ml Wasser, das sich in einem Behälter befindet, wird so viel einer 0,1 %igen acetonischen Lösung des Wirkstoffes pipettiert, dass eine Konzentration von 400 ppm erhalten wird. Nach Verdunsten des Acetons wird der Behälter mit 30 bis 40 2-tägigen Aëdes-Larven beschickt. Nach 2 und 7 Tagen wird die Mortalität geprüft.

Verbindungen der Formel I gemäss den vorstehenden Beispielen 3, 4 und 5 zeigen gute Wirkung im obigen Test.

Beispiel 17: Formulierungen für Wirkstoffe der Formel I resp. Kombinationen dieser Wirkstoffe mit andern Insektiziden oder Akariziden (% = Gewichtsprozent):

| 1. Spritzpulver | a) | b) | c) |
|---|---|---|---|
| Wirkstoff oder Wirkstoff-kombination | 25 % | 50 % | 75 % |
| Na-Ligninsulfonat | 5 % | 5 % | - |
| Na-Laurylsulfat | 3 % | - | 5 % |
| Na-Diisobutylnaphthalinsulfonat | - | 6 % | 10 % |
| Octylphenolpolyäthylenglykoläther (7-8 Mol AeO) | - | 2 % | - |
| Hochdisperse Kieselsäure | 5 % | 10 % | 10 % |
| Kaolin | 62 % | 27 % | - |

Der Wirkstoff oder die Wirkstoffkombination wird mit den Zusatzstoffen vermischt und in einer geeigneten Mühle gut vermahlen.

Man erhält Spritzpulver, die sich mit Wasser zu Suspensionen jeder gewünschten Konzentration verdünnen lassen.

2. Emulsions-Konzentrat

Wirkstoff oder Wirkstoffkombination 10 %
Octylphenolpolyäthylenglykoläther (4-5 Mol AeO) 3 %
Ca-Dodecylbenzolsulfonat 3 %
Ricinusölpolyglykoläther (36 Mol AeO) 4 %
Cyclohexanon 30 %
Xylolgemisch 50 %

Aus diesem Konzentrat können durch Verdünnen mit Wasser Emulsionen jeder gewünschten Konzentration hergestellt werden.

| 3. Stäubemittel | a) | b) |
|---|---|---|
| Wirkstoff oder Wirkstoffkombination | 5 % | 8 % |
| Talkum | 95 % | - |
| Kaolin | - | 92 % |

Man erhält anwendungsfertige Stäubemittel, indem der Wirkstoff mit dem Träger vermischt und auf einer geeigneten Mühle vermahlen wird.

4. Extruder-Granulat

Wirkstoff oder Wirkstoffkombination 10 %
Na-Ligninsulfonat 2 %
Carboxymethylcellulose 1 %
Kaolin 87 %

Der Wirkstoff wird mit den Zusatzstoffen vermischt, vermahlen und mit Wasser angefeuchtet. Dieses Gemisch wird extrudiert, granuliert und anschliessend im Luftstrom getrocknet.

5. Umhüllungs-Granulat

Wirkstoff oder Wirkstoffkombination 3 %
Polyäthylenglykol (MG 200) 3 %
Kaolin 94 %

Der fein gemahlene Wirkstoff oder die Wirkstoffkombination wird in einem Mischer auf das mit Polyäthylenglykol angefeuchtete Kaolin gleichmässig aufgetragen. Auf diese Weise erhält man staubfreie Umhüllungs-Granulate.

6. Suspensions-Konzentrat

Wirkstoff oder Wirkstoffkombination 40 %
Aethylenglykol 10 %
Nonylphenolpolyäthylenglykoläther (15 Mol AeO) 6 %
Na-Ligninsulfonat 10 %
Carboxymethylcellulose 1 %
37%ige wässrige Formaldehyd-Lösung 0,2 %

Silikonöl in Form einer 75%igen wässrigen Emulsion 0,8 %
Wasser 32 %
Der fein gemahlene Wirkstoff oder die Wirkstoffkombination wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

**Patentansprüche**

1. Verbindungen der Formel I

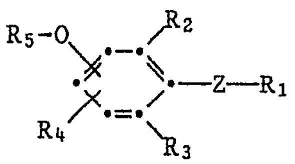

(I),

worin

Z 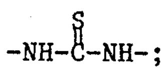 ; 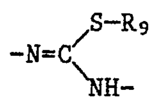 oder $-N{=}C{=}N-$;

$R_1$ $C_1$-$C_{12}$-Alkyl; $C_3$-$C_{10}$-Alkenyl; $C_3$-$C_{10}$-Alkinyl; $C_3$-$C_8$-Cycloalkyl; mit 1 oder 2 $C_3$-$C_6$-Cycloalkylresten substituiertes $C_1$-$C_3$-Alkyl; Alkoxyalkyl mit insgesamt 3-10 C-Atomen, halogensubstituiertes $C_1$-$C_{12}$-Alkyl; polycyclisches $C_7$-$C_{10}$-Cycloalkyl; oder Phenyl-$C_1$-$C_5$-alkyl, gegebenenfalls im Phenylteil bis zu zweifach gleich oder verschieden mit Halogen, Methoxy, Ethoxy oder $C_1$-$C_5$-Alkyl substituiert;
$R_2$ Wasserstoff; oder $C_1$-$C_5$-Alkyl;
$R_3$ $C_1$-$C_5$-Alkyl; oder $C_3$-$C_6$-Cycloalkyl;
$R_4$ Wasserstoff; oder $C_1$-$C_4$-Alkyl;
$R_5$ einen 5-gliedrigen heterozyclischen Rest mit 1-4 Heteroatomen der Gruppe N,O,S, welcher bis zu dreifach an den Kohlenstoff-bzw. Stickstoffatomen durch die Reste $R_6$, $R_7$, $R_8$ substituiert sein kann; oder einen benzokondensierten 5-gliedrigen heterozyclischen Rest mit 1-2 Heteroatomen der Gruppe N,O,S, welcher bis zu dreifach an den Kohlenstoff- bzw. Stickstoffatomen durch die Reste $R_6$, $R_7$, $R_8$ substituiert sein kann;
$R_6$ und $R_7$ unabhängig voneinander Wasserstoff; Halogen; $C_1$-$C_4$-Alkyl; halogensubstituiertes $C_1$-$C_4$-Alkyl; $C_1$-$C_4$-Alkoxy; halogensubstituiertes $C_1$-$C_4$-Alkoxy; Phenyl; oder $C_1$-$C_4$-Alkylthio;
$R_8$ Wasserstoff; oder $C_1$-$C_4$-Alkyl;
$R_9$ $C_1$-$C_5$-Alkyl; $C_2$-$C_5$-Alkenyl; $C_3$-$C_4$-Alkinyl; Alkoxyalkyl mit insgesamt bis zu 10 C-Atomen; oder Alkylthioalkyl mit insgesamt bis zu 10 C-Atomen
bedeuten,
sowie deren Salze mit Säuren und Basen.

2. Verbindungen der Formel I nach Anspruch 1, worin $R_5$ einen heterozyklischen Rest

$Q_1$ = 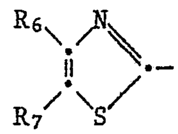 ; $Q_2$ = 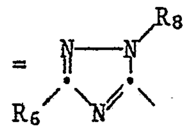 ; $Q_3$ = 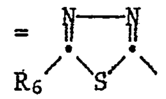 ;

$Q_4$ = 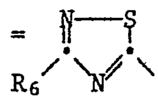 ; $Q_5$ = 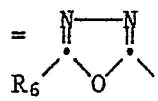 ; $Q_6$ = 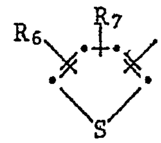 ;

$Q_7$ = 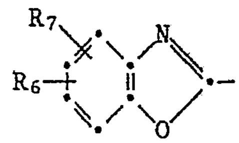 $Q_8$ = 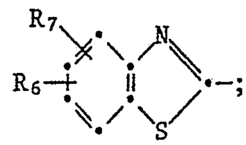 ; $Q_9$ = 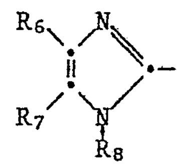 ;

$Q_{10}$ = 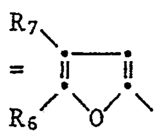 ; $Q_{11}$ = 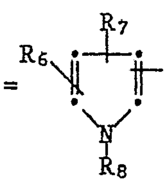 ; $Q_{12}$ = 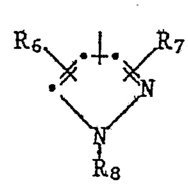 ;

$Q_{13}$ = 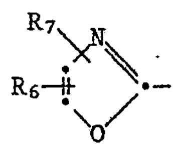 ; $Q_{14}$ = 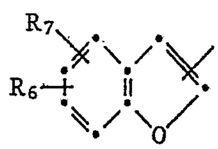 ; $Q_{15}$ = 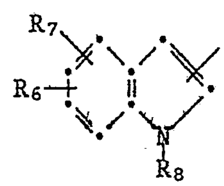 ;

$Q_{16}$ = 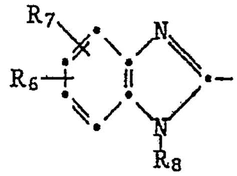 ; oder $Q_{17}$ = 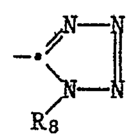

3. Verbindungen der Formel I, nach Anspruch 1 oder 2, worin die Gruppe $R_5O$ in para Stellung zur Gruppe Z steht und

Z 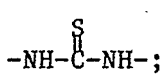 ; 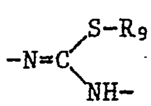 oder $-N{=}C{=}N-$;

$R_1$ $C_3$-$C_7$-Alkyl; Cyclopentyl; Cyclohexyl; (Di-cyclopropyl)-methyl; oder Alkoxyalkyl mit bis zu 4 C-Atomen;
$R_2$ und $R_3$ unabhängig voneinander $C_1$-$C_3$-Alkyl;
$R_4$ Wasserstoff;
$R_5$ einen heterozyclischen Rest

$Q_1$ = 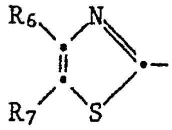 ; $Q_2$ = 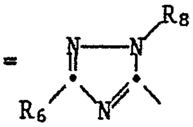 ;

$Q_3$ = 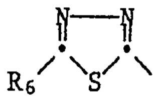 ; $Q_4$ = 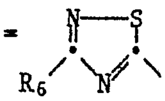 ;

$Q_5$ = 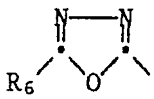 ; $Q_6$ = 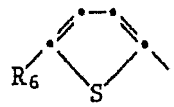 ;

$Q_7$ = 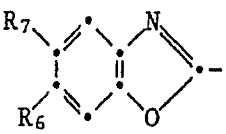 ; $Q_8$ = 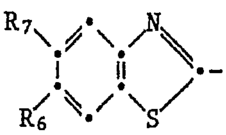 ; oder

$Q_9$ = 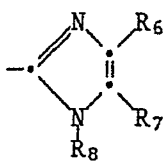 ;

$R_6$ Wasserstoff; Chlor; Brom; $C_1$-$C_4$-Alkyl; $C_1$-$C_3$-Perfluoralkyl; oder Phenyl;
$R_7$ Wasserstoff; Chlor; Brom; oder $C_1$-$C_4$-Alkyl;
$R_8$ Wasserstoff; oder $C_1$-$C_3$-Alkyl;
$R_9$ Methyl; oder Aethyl bedeuten;
sowie deren Salze mit Halogenwasserstoffsäuren.

4. Verbindungen der Formel I nach Anspruch 1 oder 2, worin die Gruppe $R_5O$ in para Stellung zur Gruppe Z steht;

Z 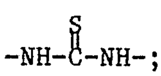 ; 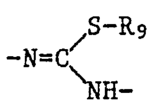 oder $-N=C=N-$;

$R_1$ tert.-Butyl; tert.-Pentyl; $C_5$-$C_6$-Cycloalkyl; oder 3-Methoxypropyl;
$R_2$ und $R_3$ unabhängig voneinander $C_1$-$C_3$-Alkyl;
$R_4$ Wasserstoff;
$R_5$ einen heterozyclischen Rest

$Q_1$ = 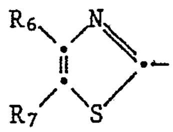 ; $Q_2$ = 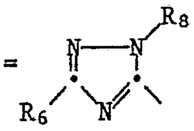 ;

$Q_3$ = 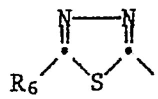 ; $Q_4$ = 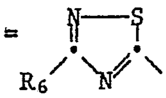 ; oder

$Q_5$ = 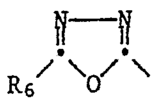 ;

$R_6$ $C_1$-$C_4$-Alkyl; Chlor; Brom; oder $C_1$-$C_3$-Perfluoralkyl;
$R_7$ Wasserstoff; Chlor; Brom; oder $C_1$-$C_4$-Alkyl;
$R_8$ $C_1$-$C_3$-Alkyl;
$R_9$ Methyl; oder Aethyl;
bedeuten,
sowie deren Salze mit Halogenwasserstoffsäuren.

5. Thioharnstoffe der Formel Ia

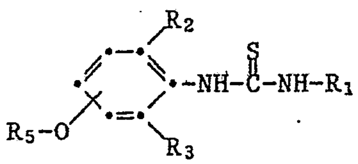 (Ia),

nach einem der Ansprüche 1 bis 4, worin
$R_1$ tert.-Butyl; iso-Propyl; Cyclopentyl; Cyclohexyl; (Di-cyclopropyl)methyl; 2,4-Dimethylpentan-3-yl; oder tert.-Pentyl;
$R_2$ und $R_3$ unabhängig voneinander Methyl; oder Ethyl; oder iso-Propyl;
$R_5$ einen heterozyclischen Rest

$Q_1$ = 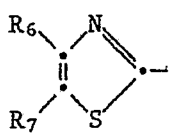 ; $Q_2$ = 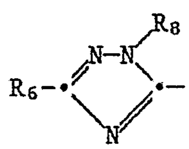 ;

$Q_3$ = 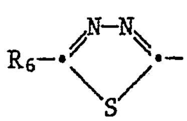 ; oder $Q_4$ = 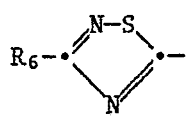 ;

$R_6$ Chlor; Brom; $CF_3$; $C_2F_5$; Methyl; oder tert.-Butyl;
$R_7$ Wasserstoff; Brom; Chlor; oder Methyl;
$R_8$ Methyl; oder iso-Propyl;
bedeuten.

6. Isothioharnstoffe der Formel Ib

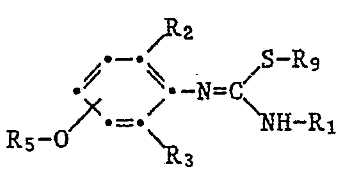 (Ib),

nach einem der Ansprüche 1 bis 4, worin

$R_1$tert.-Butyl; iso-Propyl; Cyclopentyl; Cyclohexyl; tert.-Pentyl; 2,4-Dimethylpentan-3-yl; oder (Di-cyclopropyl)methyl;
$R_2$ und $R_3$ unabhängig voneinander Methyl oder Aethyl; oder iso-Propyl;
$R_5$ einen Rest

$Q_1$ = 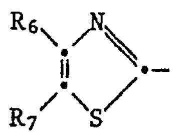 ; 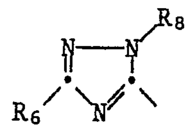 oder $Q_4$ = 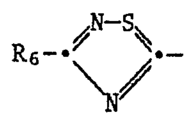

$R_6$ Chlor; Brom; Methyl; Pentafluoräthyl; oder Trifluormethyl;
$R_7$ Wasserstoff; Brom; Chlor; oder Methyl;
$R_8$ Methyl; oder iso-Propyl und
$R_9$ Methyl; oder Aethyl
bedeuten; sowie deren Salze mit Halogenwasserstoffsäuren.

7. Carbodiimide der Formel Ic

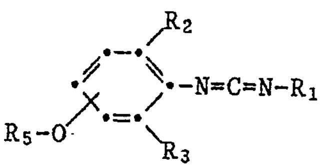 (Ic)

nach einem der Ansprüche 1 bis 4, worin
$R_1$ tert.-Butyl; Cyclopentyl; oder tert.-Pentyl;
$R_2$ und $R_3$ unabhängig voneinander Methyl; Aethyl; oder iso-Propyl;
$R_5$ einen heterozyclischen Rest

$Q_1$ = 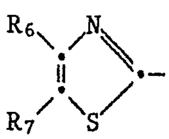 ; $Q_2$ = 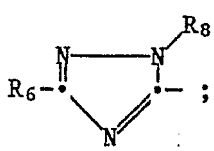 ;

$Q_3$ = 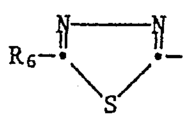 ; oder $Q_4$ = 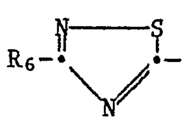 ;

$R_6$ Chlor; Brom; Methyl; tert.-Butyl; Pentafluroäthyl; oder Trifluormethyl;
$R_7$ Chlor; Brom; oder Methyl und
$R_8$ Methyl; oder Isopropyl;
bedeuten.

8. Verbindungen der Formeln Ia, Ib oder Ic gemäss Anspruch 5, 6 bzw. 7, dadurch gekennzeichnet, dass die Gruppe $R_5$-O- in para-Stellung steht.

9. Verbindung gemäss Anspruch 6 der Formel

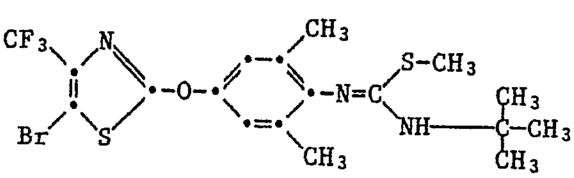 .

10. Verbindung gemäss Anspruch 6 der Formel

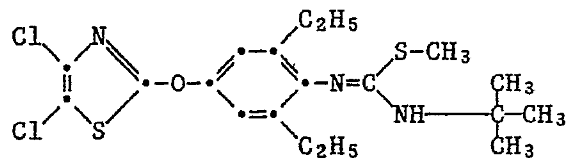

11. Verbindung gemäss Anspruch 7 der Formel

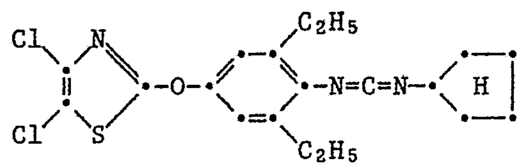

12. Verbindung gemäss Anspruch 7 der Formel

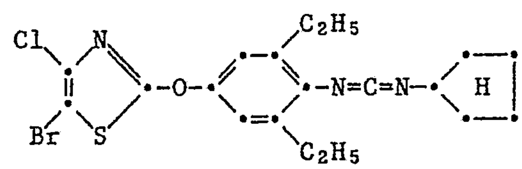

13. Verbindung gemäss Anspruch 5 der Formel

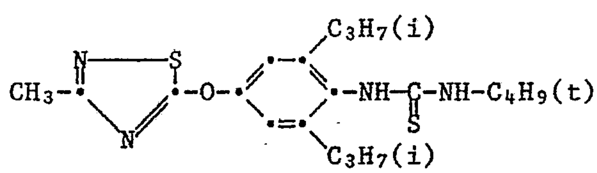

14. Verbindung gemäss Anspruch 6 der Formel

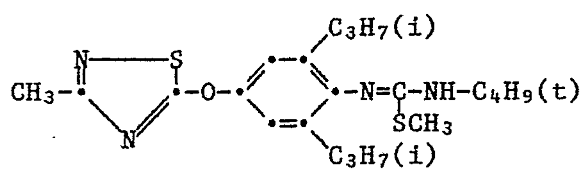

15. Verbindung gemäss Anspruch 5 der Formel

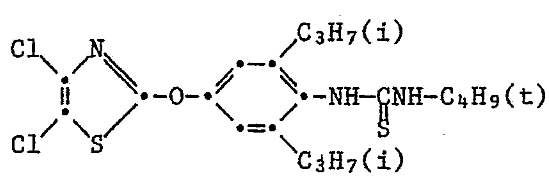

16. Verbindung gemäss Anspruch 6 der Formel

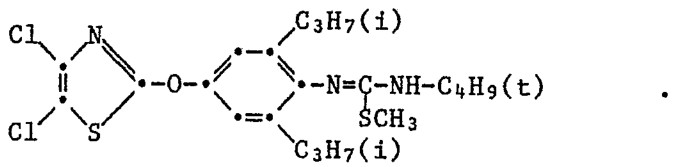

17. Verbindung gemäss Anspruch 7 der Formel

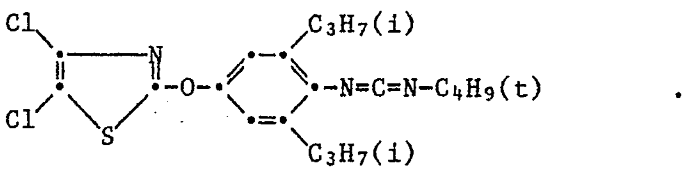

18. Verbindung gemäss Anspruch 6 der Formel

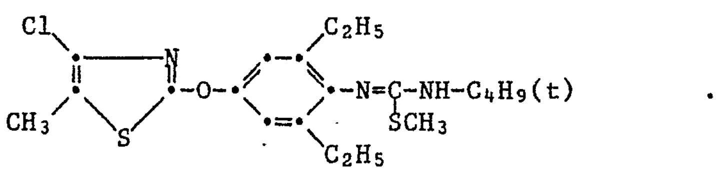

19. Verfahren zur Herstllung von Verbindungen der Formel I nach einem der Ansprüche 1 bis 18, dadurch gekennzeichnet, dass man

a) ein Amin der Formel VI mit einem Isothiocyanat der Formel II

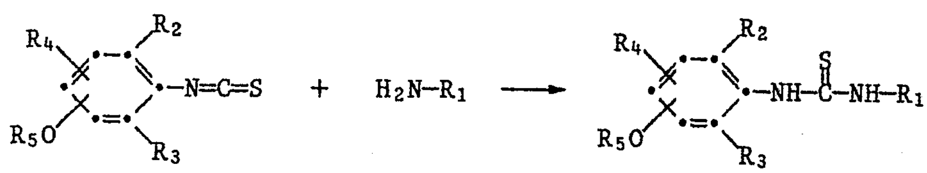

(II) (VI) (Ia)

zu einem Thioharnstoff der Formel Ia oder

b) einen Thioharnstoff der Formel Ia mit einer Verbindung der Formel VII

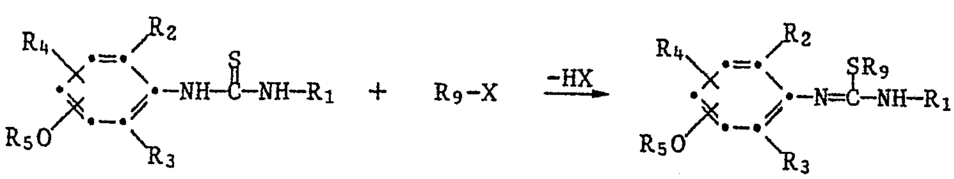


(Ia) (VII) (Ib)

zu einem Isothioharnstoff der Formel Ib oder

c) einen Thioharnstoff der Formel Ia unter Abspaltung von Schwefelwasserstoff mittels geeigneter Reagentien, wie etwa 2-Chlor-1-methyl-pyridinium-Iodid,

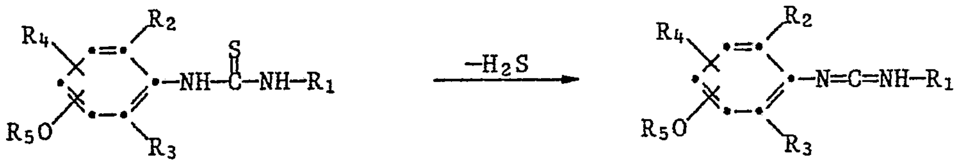


(Ia) (Ic)

zu einem Carbodiimid der Formel Ic umsetzt, wobei die Substituenten $R_1$ bis $R_9$ und Z gemäss den Ansprüchen 1 bis 7 definiert sind und X für eine unter den Reaktionsbedingungen abspaltbare Abgangsgruppe, wie Halogen, gegebenenfalls halogenierte oder alkylierte Sulfonsäureester oder Dialkylsulfat, steht.

20. Schädlingsbekämpfungsmittel, enthaltend als Wirkstoff mindestens eine Verbindung der Formel I gemäss den Ansprüchen 1 bis 18 neben Hilfs- und/oder Trägerstoffen.

21. Verwendung einer Verbindung gemäss den Ansprüchen 1 bis 18 zur Bekämpfung von Insekten und Vertretern der Ordnung Akarina an Tieren und Pflanzen.

22. Verwendung gemäss Anspruch 21 zur Bekämpfung von Zecken und Milben.

23. Verwendung gemäss Anspruch 22 zur Bekämpfung von pflanzenschädigenden Milben.

24. Verwendung gemäss Anspruch 21 zur Bekämpfung von pflanzenschädigenden Insekten.

25. Verfahren zur Bekämpfung von Insekten und Vertretern der Ordnung Akarina, dadurch gekennzeichnet, dass man die Schädlinge bzw. deren verschiedene Entwicklungsstadien und/oder ihren Aufenthaltsort mit einer pestizid wirksamen Menge einer Verbindung gemäss einem der Ansprüche 1 bis 18 oder mit einem Mittel enthaltend neben Zusatz-und Trägerstoffen eine pestizid wirksame Menge dieser Verbindung, in Kontakt bringt oder behandelt.

26. Isothiocyanate der Formel II

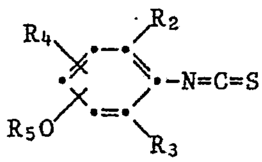

(II),

worin die Substituenten $R_2$ bis $R_5$ gemäss den Ansprüchen 1 bis 8 definiert sind.

27. Isothiocyanate der Formel II gemäss Anspruch 26, worin $R_4$ Wasserstoff bedeutet und die Gruppe $R_5O$- in para-Stellung zur Gruppe $-N=C=S$ steht.

28. Verfahren zur Herstellung von Isothiocyanaten der Formel II gemäss Anspruch 26 oder 27, dadurch gekennzeichnet, dass man ein Anilin der Formel III, worin $R_2$ bis $R_5$ die unter Anspruch 1 bis 8 angegebenen Bedeutungen haben,

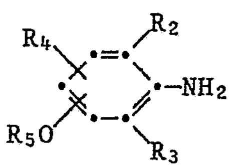

(III)

mit Thiophosgen kondensiert.

29. Aniline der Formel III

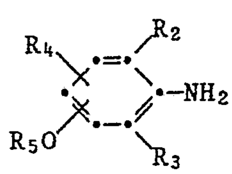

(III),

worin die Substituenten $R_2$ bis $R_5$ gemäss Anspruch 1 bis 8 definiert sind.

30. Anilin der Formel III gemäss Anspruch 29, worin $R_4$ Wasserstoff bedeutet und der Substituent $R_5$ in para-Stellung zur Aminogruppe steht.

31. Verfahren zur Herstellung von Anilinen der Formel III gemäss Anspruch 29 oder 30, dadurch

gekennzeichnet, dass man ein Aminophenol der Formel IV in Gegenwart von basischen Substanzen mit einer Verbindung der Formel V,

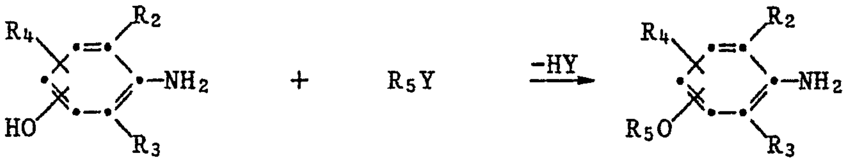

(IV) (V) (III)

worin Y für eine unter den Reaktionsbedingungen abspaltbare Abgangsgruppe, wie Halogen oder $C_1$-$C_4$-Alkylsulfonyl, steht, umsetzt.

Europäisches Patentamt

# EUROPÄISCHER RECHERCHENBERICHT

Nummer der Anmeldung

EP 87 81 0498

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| Y | EP-A-0 177 710 (BAYER)<br>* Seiten 1-5; Seite 11, Formelen IIa,b,c; Seiten 28-33 * | 1-6,13, 15,19-31 | C 07 D 277/34<br>C 07 D 233/70<br>C 07 D 285/12<br>C 07 D 249/12<br>C 07 D 271/10<br>C 07 D 285/08<br>C 07 D 277/68<br>A 01 N 43/78<br>A 01 N 43/76<br>A 01 N 43/82<br>A 01 N 43/653 |
| A | EP-A-0 192 180 (CIBA-GEIGY) | | |
| A | EP-A-0 131 973 (INSTITUTO DE ANGELI) | | |
| A | EP-A-0 088 008 (SHIONOGI) | | |

**RECHERCHIERTE SACHGEBIETE (Int. Cl.4)**

C 07 D 277/00
C 07 D 233/00
C 07 D 285/00
C 07 D 249/00
C 07 D 271/00
A 01 N 43/00

Der vorliegende Recherchenbericht wurde für alle Patentansprüche erstellt

| Recherchenort | Abschlußdatum der Recherche | Prüfer |
|---|---|---|
| DEN HAAG | 09-12-1987 | DE BUYSER I.A.F. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)